# EUROPEAN PATENT SPECIFICATION

(11) **EP 4 583 796 B1**
(45) Date of publication and mention of the grant of the patent: **11.11.2026**
(21) Application number: 23801080.5
(22) Date of filing: 03.10.2023
(51) Int. Cl.: A61B 17/34, A61B 5/02, A61M 31/00, A61B 17/11, A61B 17/22, A61M 25/00

(54) **DEVICES AND METHODS FOR CONFIRMING TISSUE CAPTURE FOR A SHUNT DEVICE**
VORRICHTUNGEN UND VERFAHREN ZUR BESTÄTIGUNG DER GEWEBEERFASSUNG FÜR EINE SHUNT-VORRICHTUNG
DISPOSITIFS ET PROCÉDÉS DE CONFIRMATION DE CAPTURE DE TISSU POUR DISPOSITIF DE DÉRIVATION

(30) Priority: 03.10.2022 US 202263378170 P
(43) Date of publication of application: 16.07.2025
(73) Proprietor: Edwards Lifesciences Corporation, Irvine, CA 92614 (US)
(72) Inventor: JAWITZ, Morgan, Alex, Torrance, California 90505 (US); QUACH, Lan, T., Irvine, California 92614 (US); KWON, Yoon, Hee, Mission Viejom, California 92691 (US); ALCANTAR CHAVEZ, Sandra, Ahide, Irvine, California 92614 (US); POOL, Scott, Louis, Irvine, California 92614 (US); CROFTON, Kirsty, Elizabeth, Irvine, California 92614 (US); SOLIS, Eliazib, Garcia, Irvine, California 92614 (US)
(74) Representative: Venner, Julia Ann
(86) International application number: PCT/US2023/034385
(87) International publication number: WO 2024/076578

(56) References cited:
- WO-A1-2014/150106
- US-A1- 2005 065 469
- US-A1- 2005 277 965
- US-A1- 2016 151 615
- US-A1- 2019 134 350
- US-A1- 2022 273 279
- US-B1- 6 464 665
- US-B1- 6 726 677

## Description

This application claims the benefit of U.S. Provisional Application No. 63/378,170, filed October 3, 2022, and entitled "DEVICES AND METHODS FOR CONFIRMING TISSUE CAPTURE FOR A SHUNT DEVICE".

### BACKGROUND

The present disclosure relates generally to implantable devices and more specifically to confirming placement of cardiovascular shunt devices.

Shunt devices can be positioned in the heart to shunt blood between the left atrium and the right atrium to reduce pressure in the left atrium. The left atrium can experience elevated pressure due to abnormal heart conditions caused by age and/or disease. For example, shunt devices can be used to treat patients with heart failure (also known as congestive heart failure). Shunt devices can be positioned in the septal wall between the left atrium and the right atrium to shunt blood from the left atrium into the right atrium, thus reducing the pressure in the left atrium.

WO 2014/150106 A1 discloses a placement catheter for an interatrial pressure vent whereby the catheter has a center lumen for injecting contrast liquid.

### SUMMARY

The invention is defined by claims 1, 11, 13, 14 and 15. Further embodiments of the invention are defined by the dependent claims.

A contrast delivery system for determining tissue capture of a shunt device includes a catheter including a lumen having a distal end, an exit hole connecting the lumen to an exterior surface of the catheter, and a side opening adjacent to the distal end of the lumen. The contrast delivery system further includes a tube slidably receivable in the lumen and configured to deliver a contrast agent, the tube including a closed distal terminal end, a first distal hole, and a proximal hole. The first distal hole and proximal hole are spaced along a length of the tube, wherein the first distal hole is disposed between the proximal hole and the closed distal terminal end. The tube is configured to slide in the lumen between a plurality of positions. The first distal hole is aligned with the exit hole of the catheter and open to allow flow of the contrast agent through first distal hole and the exit hole when the tube is in a first position. The first distal hole is positioned in the side opening and open to allow flow of the contrast agent through the first distal hole and the side opening when the tube is in a second position.

A contrast delivery system for determining tissue capture of a shunt device includes a catheter including a lumen having a distal end, an exit hole connecting the lumen to an exterior surface of the catheter, and a side opening spaced from the exit hole along a length of the catheter, the side opening disposed adjacent to and open to the distal end of the lumen. A tube is received in the lumen and configured to deliver a contrast agent, the tube including a plurality of holes spaced along a length of the tube. The tube is configured to be advanced and retracted in the lumen to align one or more holes of the plurality of holes with each of the exit hole and the side opening.

A method for determining tissue capture of a shunt device, the shunt device having a flow tube, proximal arm, and distal arm, the proximal arm and distal arm configured to capture tissue therebetween. The method includes positioning, with a catheter, the shunt device within a human body, and positioning a tube in one of a plurality of positions in the catheter. The tube includes a closed distal terminal end, a first distal hole, and a proximal hole, the first distal hole and proximal hole spaced along a length of the tube. The first distal hole is positioned between the proximal hole and the closed distal terminal end. The method further includes injecting a contrast agent through the first distal hole when the tube is in a first position, wherein the proximal hole is closed when the tube is in the first position, advancing or retracting the tube to a second position, and injecting the contrast agent through the first distal hole and the proximal hole when the tube is in a second position.

A contrast delivery system for determining tissue capture of a shunt device, the shunt device having a flow tube, proximal arm, and distal arm, the proximal arm and distal arm configured to capture tissue therebetween. The contrast delivery system includes a catheter including a lumen, a channel extending along a length of the catheter and open to an outer surface of the catheter, and a feed port connecting the lumen to the channel, wherein the lumen, channel, and feed port are configured to deliver a contrast agent. The catheter is positioned through the flow tube and wherein the flow tube is substantially aligned with the channel.

A method for determining tissue capture of a shunt device, the shunt device having a flow tube, proximal arm, and distal arm, the proximal arm and distal arm configured to capture tissue therebetween. The method includes positioning, with a catheter, the shunt device within a human body. The catheter includes a lumen, a channel extending along a length of the catheter and open to an outer surface of the catheter, and a feed port connecting the lumen to the channel. The method further includes injecting a contrast agent through lumen, feed port, and channel of the catheter and through a side of the flow tube of the shunt device toward ends of the proximal and distal arms.

A contrast delivery system for determining tissue capture of a shunt device, the shunt device having a flow tube, proximal arm, and distal arm, the proximal arm and distal arm configured to capture tissue therebetween. The contrast delivery system includes a catheter configured to deliver the shunt device, the catheter including a lumen, and a side opening. The contrast delivery system further includes a tube received in the lumen and configured to deliver a contrast agent, the tube comprising an inflatable balloon on a distal end, wherein the balloon is disposed adjacent to the side opening and an exterior surface of the flow tube of the shunt device.

A method of determining tissue capture of a shunt device, the shunt device having a flow tube, proximal arm, and distal arm, the proximal arm and distal arm configured to capture tissue therebetween. The method includes positioning a catheter in an opening in a tissue wall, and delivering a shunt device to the tissue wall, wherein the distal arm is configured to be seated on a first side of the tissue wall and the proximal arm is configured to be seated on a second side of the tissue wall. The method further includes injecting a contrast agent into an inflatable balloon following delivery of the shunt device, wherein the inflatable balloon is disposed along an exterior surface of the flow tube of the shunt device on a catheter and wherein injecting the contrast agent causes the inflatable balloon to expand. The method additionally includes imaging a region of a body including the shunt device and inflatable balloon following injection of the contrast agent to visualize a shape of the expanded inflatable balloon.

A contrast delivery system for determining tissue capture of a shunt device, the shunt device having a flow tube, proximal arm, and distal arm, the proximal arm and distal arm configured to capture tissue therebetween. The contrast delivery system includes a catheter configured to deliver the shunt device, the catheter including a lumen, and a side opening. The contrast delivery system further includes a tube receivable in the lumen and configured to deliver a contrast agent. The tube includes a closed distal terminal end, and a slit opening extending along a length of the tube, wherein a portion of the tube including the slit opening is disposable adjacent to an exterior surface of the flow tube of the shunt device and wherein the slit opening faces away from the exterior surface of the flow tube.

A method of determining tissue capture of a shunt device, the shunt device having a flow tube, proximal arm, and distal arm, the proximal arm and distal arm configured to capture tissue therebetween. The method includes positioning a catheter in an opening in a tissue wall, and delivering a shunt device to the tissue wall, wherein the distal arm is configured to be seated on a first side of the tissue wall and the proximal arm is configured to be seated on a second side of the tissue wall. The method further includes injecting a contrast agent through a slit opening of a tube following delivery of the shunt device, wherein the slit opening extends a length of the tube and wherein a portion of the tube having the slit opening is disposed adjacent to an exterior surface of the flow tube on a catheter with the slit opening facing away from the exterior surface. The method additionally includes imaging a region of a body including the shunt device and the tube to visualize a contrast agent delivered from the slit opening.

### BRIEF DESCRIPTION OF THE DRAWINGS

### ANATOMY OF HEART H AND VASCULATURE V

FIG. 1 is a schematic diagram of a heart and vasculature.
FIG. 2 is a schematic cross-sectional view of the heart.

### SHUNT DEVICES 100 AND 100'

FIG. 3A is a perspective view of a shunt device.
FIG. 3B is a side view of the shunt device.
FIG. 4 is a perspective view of the shunt device in a collapsed configuration.
FIG. 5 is a perspective view of a shunt device including a sensor.

### DELIVERY CATHETER 200

FIG. 6 is a side view of a delivery catheter.
FIG. 7A is a side view of a distal portion of the delivery catheter in a sheathed state.
FIG. 7B is a side view of the distal portion of the delivery catheter in an unsheathed state.

### DELIVERY METHOD 300

FIG. 8A is a flow chart showing steps for creating a puncture in a tissue wall between a coronary sinus and a left atrium.
FIG. 8B is a flow chart showing steps for implanting a shunt device in the tissue wall between the coronary sinus and the left atrium.
FIGS. 9A-9Q are schematic views showing the steps for implanting a shunt device in the tissue wall between the coronary sinus and the left atrium.
FIG. 10A is a simplified perspective view of the shunt device properly seated between the left atrium and coronary sinus.
FIG. 10B is a simplified perspective view of the shunt device improperly seated between the left atrium and coronary sinus.
FIG. 10C is a simplified perspective view of the shunt device embolized in a left atrium.

### DELIVERY DEVICE 1500

FIG. 11A is a cross-sectional view of a shunt delivery device having an adjustable contrast tube.
FIG.11B is a simplified perspective view of the shunt delivery device of FIG. 11A with the adjustable contrast tube in a first position.
FIG. 11C is a simplified perspective view of the shunt delivery device of FIG. 11A with the adjustable contrast tube in a second position.
FIG. 11D is a simplified perspective view of the shunt delivery device of FIG. 11A with the adjustable contrast tube in a third position.
FIG. 11E is side view of the shunt delivery device of FIG. 11A.
FIG. 11F is a bottom view of shunt delivery device of FIG. 11A.
FIG. 11G is a side view of the adjustable contrast tube of FIG. 11A.
FIG. 11H is a bottom view of the adjustable contrast tube of FIG. 11A.FIG. 12A is a perspective view of a shunt delivery device having an adjustable contrast tube with a curved distal tip.
FIG. 12B is a simplified perspective view of the shunt delivery device of FIG. 12A with the adjustable contrast tube in a first position.
FIG. 12C is a simplified perspective view of the shunt delivery device of FIG. 12A with the adjustable contrast tube in a second position.
FIG. 12D is a simplified perspective view of the shunt delivery device of FIG. 12A with the adjustable contrast tube in a third position.
FIG. 13 is a side view of one example of the adjustable contrast tube of FIGS. 12A-12D.
FIG. 14 is a side view of one example of the adjustable contrast tube of FIGS. 12A-12D.

### DELIVERY DEVICE 1600

FIG. 15 is a perspective view of a shunt delivery device having a contrast delivery channel.
FIG. 16 is a side view of the shunt delivery device of FIG. 15 with contrast delivery channel.

### DELIVERY DEVICE 1700

FIG. 17 is a perspective view of a shunt delivery device having contrast tube with a delivery slot positioned outside a central flow tube of the shunt device.
FIG. 18 is a simplified view of the contrast tube of FIG. 17 illustrating delivery of a contrast agent.

### DELIVERY DEVICE 1800

FIG. 19A is a perspective view of a shunt delivery device having contrast tube with an inflated balloon positioned outside a central flow tube of the shunt and illustrating proper tissue capture.
FIG. 19B is a perspective view of the shunt delivery device of FIG. 19A illustrating improper tissue capture.
FIG. 19C is a perspective view of the shunt delivery device of FIG. 19A with the balloon deflated.
FIG. 20 is a perspective view of a shunt device for use with the contrast tubes of FIGS. 18 and 19A-19C.

### DETAILED DESCRIPTION

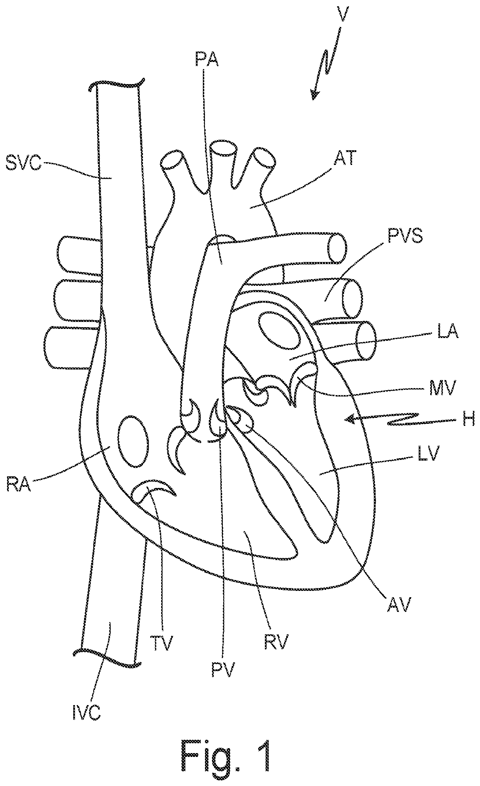

### ANATOMY OF HEART H AND VASCULATURE V (FIGS. 1-2)

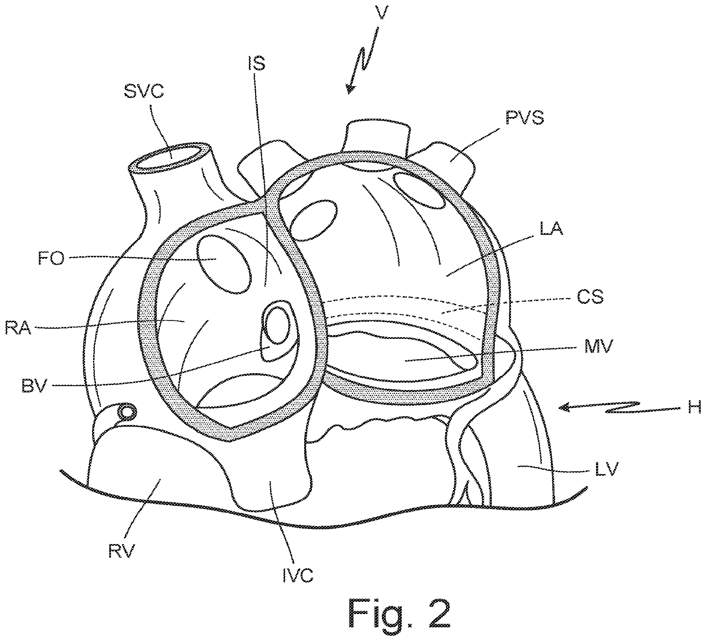

FIG. 1 is a schematic diagram of heart H and vasculature V. FIG. 2 is a cross-sectional view of heart H. FIGS. 1-2 will be described together. FIGS. 1-2 show heart H, vasculature V, right atrium RA, right ventricle RV, left atrium LA, left ventricle LV, superior vena cava SVC, inferior vena cava IVC, tricuspid valve TV (shown in FIG. 1), pulmonary valve PV (shown in FIG. 1), pulmonary artery PA (shown in FIG. 1), pulmonary veins PVS, mitral valve MV, aortic valve AV (shown in FIG. 1), aorta AT (shown in FIG. 1), coronary sinus CS (shown in FIG. 2), thebesian valve BV (shown in FIG. 2), inter-atrial septum IS (shown in FIG. 2), and fossa ovalis FO (shown in FIG. 2).

Heart H is a human heart that receives blood from and delivers blood to vasculature V. Heart H includes four chambers: right atrium RA, right ventricle RV, left atrium LA, and left ventricle LV.

The right side of heart H, including right atrium RA and right ventricle RV, receives deoxygenated blood from vasculature V and pumps the blood to the lungs. Blood flows into right atrium RA from superior vena cava SVC and inferior vena cava IVC. Right atrium RA pumps the blood through tricuspid valve TV into right ventricle RV. The blood is then pumped by right ventricle RV through pulmonary valve PV into pulmonary artery PA. The blood flows from pulmonary artery PA into arteries that delivery the deoxygenated blood to the lungs via the pulmonary circulatory system. The lungs can then oxygenate the blood.

The left side of heart H, including left atrium LA and left ventricle LV, receives the oxygenated blood from the lungs and pumps the blood to the body. Blood flows into left atrium LA from pulmonary veins PVS. Left atrium LA pumps the blood through mitral valve MV into left ventricle LV. The blood is then pumped by left ventricle LV through aortic valve AV into aorta AT. The blood flows from aorta AT into arteries that deliver the oxygenated blood to the body via the systemic circulatory system.

Blood is additionally received in right atrium RA from coronary sinus CS. Coronary sinus CS collects deoxygenated blood from the heart muscle and delivers it to right atrium RA. Thebesian valve BV is a semicircular fold of tissue at the opening of coronary sinus CS in right atrium RA. Coronary sinus CS is wrapped around heart H and runs in part along and beneath the floor of left atrium LA right above mitral valve MV, as shown in FIG. 2. Coronary sinus CS has an increasing diameter as it connects to right atrium RA.

Inter-atrial septum IS and fossa ovalis FS are also shown in FIG. 2. Inter-atrial septum IS is the wall that separates right atrium RA from left atrium LA. Fossa ovalis FS is a depression in inter-atrial septum IS in right atrium RA. At birth, a congenital structure called a foramen ovale is positioned in inter-atrial septum IS. The foramen ovale is an opening in inter-atrial septum IS that closes shortly after birth to form fossa ovalis FS. The foramen ovale serves as a functional shunt in utero, allowing blood to move from right atrium RA to left atrium LA to then be circulated through the body. This is necessary in utero, as the lungs are in a sack of fluid and do not oxygenate the blood. Rather, oxygenated blood is received from the mother. The oxygenated blood from the mother flows from the placenta into inferior vena cava IVC through the umbilical vein and the ductus venosus. The oxygenated blood moves through inferior vena cava IVC to right atrium RA. The opening of inferior vena cava IVC in right atrium RA is positioned to direct the oxygenated blood through right atrium RA and the foramen ovale into left atrium LA. Left atrium LA can then pump the oxygenated blood into left ventricle LV, which pumps the oxygenated blood to aorta AT and the systemic circulatory system. This allows the pulmonary circulatory system to be bypassed in utero. Upon birth, respiration expands the lungs, blood begins to circulate through the lungs to be oxygenated, and the foramen ovale closes to form fossa ovalis FS.

Shunt devices can be positioned in heart H to shunt blood between left atrium LA and right atrium RA. Left atrium LA can experience elevated pressure due to abnormal heart conditions. It has been hypothesized that patients with elevated pressure in left atrium LA may benefit from a reduction of pressure in left atrium LA. Shunt devices can be used in these patients to shunt blood from left atrium LA to right atrium RA to reduce the pressure of blood in left atrium LA, which reduces the systolic preload on left ventricle LV. Reducing pressure in left atrium LA further relieves back-pressure on the pulmonary circulation to reduce the risk of pulmonary edema.

For example, shunt devices can be used to treat patients with heart failure (also known as congestive heart failure). The hearts of patients with heart failure do not pump blood as well as they should. Heart failure can affect the right side and/or the left side of the heart. Diastolic heart failure (also known as heart failure with preserved ejection fraction) refers to heart failure occurring when the left ventricle is stiff (having less compliance), which makes it hard to relax appropriately and fill with blood. This leads to increased end-diastolic pressure, which causes an elevation of pressure in left atrium LA. There are very few, if any, effective treatments available for diastolic heart failure. Other examples of abnormal heart conditions that cause elevated pressure in left atrium LA are systolic dysfunction of the left ventricle and valve disease.

Septal shunt devices (also called inter-atrial shunt devices) are positioned in inter-atrial septum IS to shunt blood directly from left atrium LA to right atrium RA. Typically, septal shunt devices are positioned in fossa ovalis FS, as fossa ovalis FS is a thinner area of tissue in inter-atrial septum IS where the two atria share a common wall. If the pressure in right atrium RA exceeds the pressure in left atrium LA, septal shunt devices can allow blood to flow from right atrium RA to left atrium LA. This causes a risk of paradoxical stroke (also known as paradoxical embolism), as emboli can move from right atrium RA to left atrium LA and then into aorta AT and the systemic circulation.

Shunt devices can also be left atrium to coronary sinus shunt devices that are positioned in a tissue wall between left atrium LA and coronary sinus CS where the two structures are in close approximation. Left atrium to coronary sinus shunt devices move blood from left atrium LA into coronary sinus CS, which then delivers the blood to right atrium RA via thebesian valve BV, the natural orifice of coronary sinus CS. Coronary sinus CS acts as an additional compliance chamber when using a left atrium to coronary sinus shunt device. Left atrium to coronary sinus shunt devices further provide increased protections against paradoxical strokes, as the blood would have to flow retrograde from right atrium RA through coronary sinus CS before entering left atrium LA. Further, left atrium to coronary sinus shunt devices also provide protection against significant right atrium RA to left atrium LA shunting, as again the blood would have to flow retrograde from right atrium RA through coronary sinus CS before entering left atrium LA.

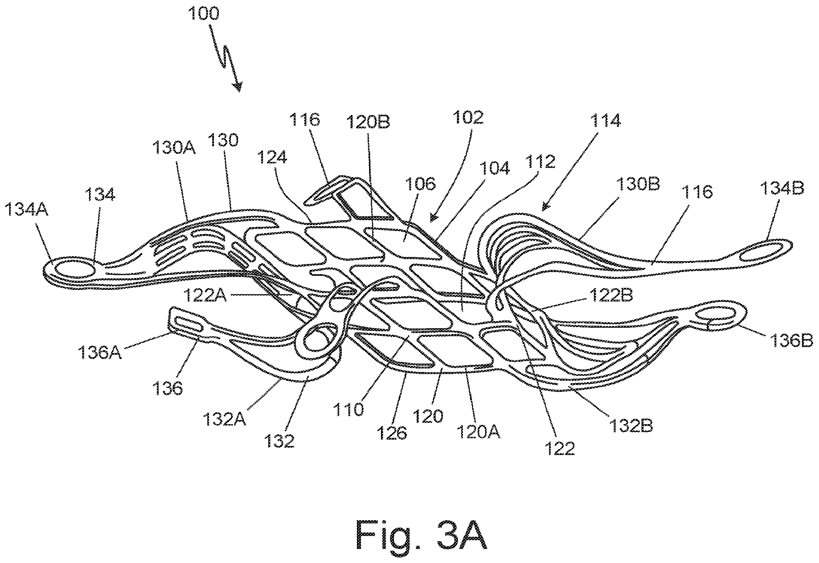

### SHUNT DEVICES 100 AND 100' (FIGS. 3A-5)

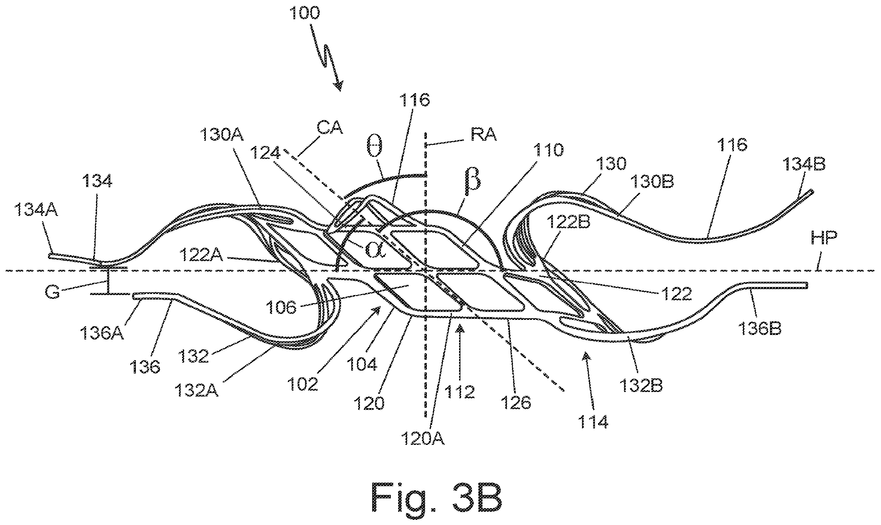

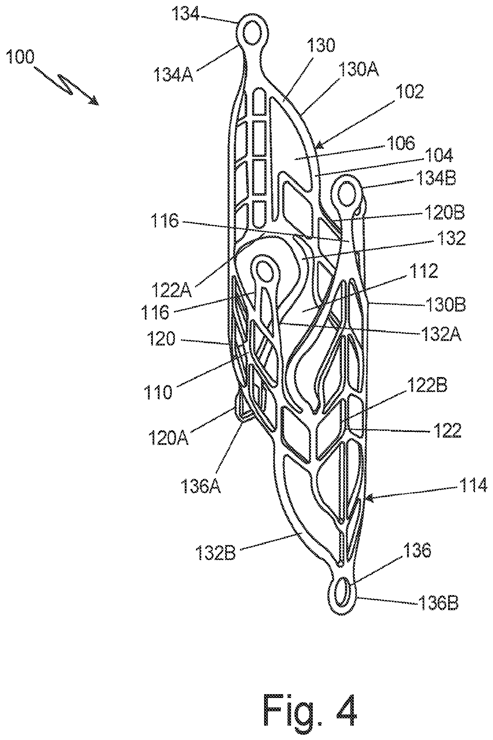

FIG. 3A is a perspective view of shunt device 100. FIG. 3B is a side view of shunt device 100. FIG. 4 is a perspective view of shunt device 100 in a collapsed configuration. FIGS. 3A, 3B, and 4 will be described together. Shunt device 100 includes body 102, which is formed of struts 104 and openings 106. Body 102 includes central flow tube 110, flow path 112, and arms 114. Shunt device 100 also includes tissue capture features 116. Central flow tube 110 has side portions 120 (including side portion 120A and side portion 120B), end portions 122 (including end portion 122A and end portion 122B), first axial end 124, and second axial end 126. Arms 114 include distal arms 130 (including distal arm 130A and distal arm 130B) and proximal arms 132 (including proximal arm 132A and proximal arm 132B). Distal arms 130 have terminal ends 134 (including terminal end 134A and terminal end 134B). Proximal arms 132 have terminal ends 136 (including terminal end 136A and terminal end 136B). FIG. 3B further shows gap G, horizontal reference plane HP, perpendicular reference axis RA, central axis CA, tilt angle θ, first angle α, and second angle β.

Shunt device 100 is a cardiovascular shunt. Shunt device 100 is shown in an expanded configuration in FIGS. 3A-3B. Shunt device 100 is formed of a super-elastic material that is capable of being compressed into a catheter for delivery into the body that can then retain its relaxed, or expanded, shape when it is released from the catheter. For example, shunt device 100 can be formed of a shape-memory material, such as nitinol (a nickel titanium alloy). Shunt device 100 is shown in a compressed configuration in FIG. 4. Upon delivery into the body, shunt device 100 will expand back to its relaxed, or expanded, shape. Shunt device 100 can be sterilized before being delivered into the body. Shunt device 100 has body 102 that is formed of interconnected struts 104. Openings 106 in body 102 are defined by struts 104. Body 102 of shunt device 100 is formed of struts 104 to increase the flexibility of shunt device 100 to enable it to be compressed and expanded.

Body 102 includes central flow tube 110 that forms a center portion of shunt device 100. Central flow tube 110 is tubular in cross-section but is formed of struts 104 and openings 106. Central flow tube 110 can be positioned in a puncture or opening in a tissue wall and hold the puncture open. Flow path 112 is an opening extending through central flow tube 110. Flow path 112 is the path through which blood flows through shunt device 100 when shunt device 100 is implanted in the body. Arms 114 extend from central flow tube 110. Arms 114 extend outward from central flow tube 110 when shunt device 100 is in an expanded configuration. Arms 114 hold shunt device 100 in position in the tissue wall when shunt device 100 is implanted in the body.

When shunt device 100 is implanted in the tissue wall between the left atrium and the coronary sinus of the heart, central flow tube 110 holds the puncture open so blood can flow from the left atrium to the coronary sinus through flow path 112. Struts 104 of central flow tube 110 form a lattice or cage of sorts that is sufficient to hold the puncture in the tissue wall open around central flow tube 110. Central flow tube 110 extends from first axial end 124 to second axial end 126. Central flow tube 110 is designed to have an axial length, as measured from first axial end 124 to second axial end 126, that approximates the thickness of the tissue wall between the left atrium and the coronary sinus. When shunt device 100 is implanted in the tissue wall between the left atrium and the coronary sinus, first axial end 124 can be facing the left atrium (i.e., a left atrial side of shunt device 100) and second axial end 126 can be facing the coronary sinus (i.e., a coronary sinus side of shunt device 100). In other examples, the orientation of first axial end 124 and second axial end 126 can be reversed.

Central flow tube 110 has side portions 120 and end portions 122. Side portion 120A and side portion 120B form opposing sides of central flow tube 110. End portion 122A and end portion 122B form opposing ends of central flow tube 110. End portion 122A and end portion 122B each extend between and connect to side portion 120A and side portion 120B to form a generally circular or oval opening that defines flow path 112. Side portions 120 and end portions 122 form a tubular lattice for central flow tube 110. Struts 104 of central flow tube 110 define openings 106 in central flow tube 110. In some examples, openings 106 can be generally parallelogram-shaped. In other examples, openings 106 can be any regular or irregular shape as desired. For example, struts 104 of side portions 120 can form an array of parallelogram-shaped openings 106 in side portions 120. Struts 104 of end portions 122 can form openings 106 in end portions 122. Struts 104 of arms 114 can form openings 106 in arms 114.

As shown in FIG. 3B, central flow tube 110 is angled with respect to horizontal reference plane HP extending through shunt device 100. Horizontal reference plane HP lies generally in the plane of the tissue wall immediately adjacent to shunt device 100 when shunt device 100 is implanted in the tissue wall. End portions 122 are similarly angled with respect to horizontal reference plane HP. Perpendicular reference axis RA, as shown in FIG. 3B, is perpendicular to horizontal reference plane HP. As shown in FIG. 3B, central axis CA is an axis through the center of central flow tube 110 and flow path 112. Central axis CA extends through central flow tube 110 at tilt angle θ with respect to perpendicular reference axis RA. Accordingly, central axis CA defines the angle or tilt of central flow tube 110 with respect to perpendicular reference axis RA (and horizontal reference plane HP). End portions 122 of central flow tube 110 extend parallel to central axis CA.

Arms 114 of shunt device 100 include two distal arms 130 and two proximal arms 132. In some examples, individual ones of distal arms 130 and/or proximal arms 132 can be formed of multiple split arm portions. Arms 114 extend outward from end portions 122 of central flow tube 110 when shunt device 100 is in an expanded configuration. Distal arm 130A is connected to and extends away from end portion 122A, and distal arm 130B is connected to and extends away from end portion 122B. Proximal arm 132A is connected to and extends away from end portion 122A, and proximal arm 132B is connected to and extends away from end portion 122B. When shunt device 100 is implanted in the tissue wall between the left atrium and the coronary sinus, distal arms 130 will be positioned in the left atrium and proximal arms 132 will be positioned in the coronary sinus. Distal arms 130 each have terminal ends 134. Specifically, distal arm 130A has terminal end 134A, and distal arm 130B has terminal end 134B. Proximal arms 132 each have terminal ends 136. Specifically, proximal arm 132A has terminal end 136A, and proximal arm 132B has terminal end 136B.

Distal arms 130 and proximal arms 132 curl outward from end walls 122. As shown in FIG. 3B, each of distal arms 130 and proximal arms 132 has a proximal portion adjacent to central flow tube 110 that forms a shallow curve or arc in a direction away from end walls 122 of central flow tube 110. Each of distal arms 130 and proximal arms 132 flattens out towards respective terminal ends 134 and 136 such that a portion of each of distal arms 130 and proximal arms 132 at or adjacent to the respective terminal end 134 or 136 is generally parallel to horizontal reference plane HP. Accordingly, an axis drawn through terminal end 134A and an axis drawn through terminal end 136B, which are approximated in FIG. 3B as axes in the plane of horizontal reference plane HP for simplicity, can each form first angle α with central axis CA through central flow tube 110. Similarly, an axis drawn through terminal end 134B, and an axis drawn through terminal end 136A, which are approximated in FIG. 3B as axes in the plane of horizontal reference plane HP for simplicity, can each form second angle β with central axis CA through central flow tube 110. Alternatively, distal arms 130 and proximal arms 132 do not flatten out and become parallel to horizontal reference plane HP but instead approach horizontal reference plane HP at an angle and/or have respective terminal ends 134 and 136 that angle away from horizontal reference plane HP. In such examples, first angle α and second angle β are approximations of the central angle for the arcs from end walls 122 to the tissue wall that each respective arm encompasses when shunt device 100 is implanted in the tissue wall. Put more simply, first angle α is the angle between central axis CA and horizontal reference plane HP, and second angle β is the supplementary angle to first angle α. In some examples, first angle α can be less than ninety degrees (<90°) and second angle β can be greater than ninety degrees (>90°). In other examples, first angle α and second angle β can be any suitable combination of angles that add to one hundred eighty degrees (180°). The difference between first angle α and second angle β (and the corresponding curvature of ones of distal arms 130 and proximal arms 132) accommodates for the tilt of central flow tube 110.

As shown in FIG. 3B, distal arm 130A and distal arm 130B extend outwards from central flow tube 110 in opposite directions parallel to horizontal reference plane HP. Distal arm 130A and distal arm 130B can be aligned with each other (i.e., oriented at 180° to each other across central flow tube 110). In some examples, distal arm 130A has a longer length than distal arm 130B. In other examples, distal arm 130A has a shorter length than distal arm 130B. In yet other examples, distal arms 130 can have similar lengths. Proximal arm 132A and proximal arm 132B extend outwards from central flow tube 110 in opposite directions parallel to horizontal reference plane HP. Proximal arm 132A and proximal arm 132B can be aligned with each other (i.e., oriented at 180° to each other across central flow tube 110). In some examples, proximal arm 132A has a shorter length than proximal arm 132B. In other examples, proximal arm 132A has a longer length than proximal arm 132B. In yet other examples, proximal arms 132 can have similar lengths. In some examples, distal arm 130A has generally the same length and shape as proximal arm 132B, and distal arm 130B has generally the same length and shape as proximal arm 132A. In other examples, each of distal arms 130 and proximal arms 132 can have different lengths and shapes, though the overall shape of each arm is similar. As such, shunt device 100 has some degree of inverse symmetry across horizontal reference plane HP, as shown in FIG. 3B.

Shunt device 100 is generally elongated longitudinally but is relatively narrow laterally. Stated another way, distal arms 130 and proximal arms 132 are not annular or circular, but rather extend outward generally in only one plane. As shown in FIG. 3B, shunt device 100 has a generally H-shape when viewing a side of shunt device 100. The elongated shape of shunt device 100 means that when compressed it elongates along a line, as shown in FIG. 4, so as to better fit within a catheter.

Terminal ends 134 of distal arms 130 and terminal ends 136 of proximal arms 132 converge towards one another. Distal arms 130 and proximal arms 132 form two pairs of arms. That is, each of distal arms 130 forms a clamping pair with a corresponding one of proximal arms 132. Distal arm 130A and proximal arm 132A form a first pair of arms extending outward from a first side of central flow tube 110, and terminal end 134A of distal arm 130A converges towards terminal end 136A of proximal arm 132A. Distal arm 130B and proximal arm 132B form a second pair of arms extending outward from a second side of central flow tube 110, and terminal end 134B of distal arm 130B converges towards terminal end 136B of proximal arm 132B. Gap G between terminal ends 134 and terminal ends 136 is sized to be slightly smaller than an approximate thickness of the tissue wall between the left atrium and the coronary sinus, or another tissue wall of interest. This allows distal arms 130 and proximal arms 132 to flex outwards and grip the tissue wall when implanted to help hold shunt device 100 in place against the tissue wall. Thus, a distance corresponding to gap G, as measured once shunt device 100 is implanted, may be slightly different between different clamping pairs of distal arms 130 and proximal arms 132 depending on anatomical variations along the particular tissue wall. Terminal ends 134 of distal arms 130 and terminal ends 136 of proximal arms 132 can also have openings or indentations that are configured to engage a delivery tool to facilitate implantation of shunt device 100, for example actuating rods of a delivery tool. Additionally, terminal ends 134 of distal arms 130 and terminal ends of proximal arms 132 can include locations for radiopaque markers to permit visualization of the positioning of shunt device 100.

When implanted in the tissue wall, distal arms 130 and proximal arms 132 are designed such that the projection of distal arms 130 and proximal arms 132 into the left atrium and the coronary sinus, respectively, is minimized. This minimizes the disruption of the natural flow patterns in the left atrium and the coronary sinus. Shunt device 100 can also be designed so that the profile of proximal arms 132 projecting into the coronary sinus is lower than the profile of distal arms 130 projecting into the left atrium to minimize disruption of the natural blood flow through the coronary sinus and to reduce the potential for proximal arms 132 to block the narrower passage of the coronary sinus.

Tissue capture features 116 can take several different forms. For example, tissue capture features 116 connected to central flow tube 110 at first axial end 124 and/or second axial end 126 can be tabs that extend outward from side portions 120. Tissue capture features 116 connected to arms 114 can be deflectable projections that extend between respective ones of arms 114 and the tissue wall to be compressed back toward the respective arm 114 when shunt device 100 is implanted in the tissue wall. Tissue capture features 116 connected to end portions 122 of central flow tube 110 can be secondary arms associated with one of arms 114. Tissue capture features 116 that are a part of arms 114 themselves can be, e.g., a lengthened portion of one of arms 114, separate split arm portions of one of arms 114, and/or interlacing arms 114. Any one or more of tissue capture features 116 can be incorporated alone or in combination on shunt device 100 to aid in anchoring shunt device 100 to the tissue wall and to prevent displacement of shunt device 100.

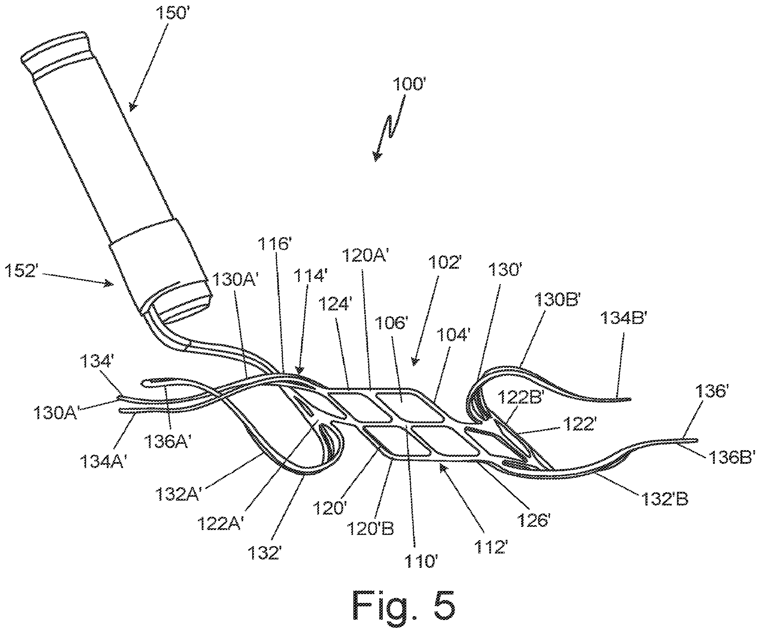

FIG. 5 is a perspective view of shunt device 100' including sensor 150'. Shunt device 100' includes body 102', which is formed of struts 104' and openings 106'. Body 102' includes central flow tube 110', flow path 112', arms 114'. Shunt device 100' also includes and tissue capture features 116'. Central flow tube 110' has side portions 120' (including side portion 120A' and side portion 120B'), end portions 122' (including end portion 122A' and end portion 122B'), first axial end 124', and second axial end 126'. Arms 114' include distal arms 130' (including distal arm 130A' and distal arm 130B') and proximal arms 132' (including proximal arm 132A' and proximal arm 132B'). Distal arms 130' have terminal ends 134' (including terminal end 134A' and terminal end 134B'). Proximal arms 132' have terminal ends 136' (including terminal end 136A' and terminal end 136B'). Shunt device 100' further includes sensor 150' and sensor attachment portion 152'.

Shunt device 100' includes a similar structure and design to shunt device 100 described above, except shunt device 100' additionally includes sensor 150' connected to sensor attachment portion 152'.

As shown in FIG. 5, sensor 150' can be attached to shunt device 100' so that sensor 150' is positioned in the left atrium when shunt device 100' is implanted in the tissue wall between the left atrium and the coronary sinus of the heart. Accordingly, sensor 150' can be attached to one of distal arms 130'. Alternatively, sensor 150' can be attached to shunt device 100' so that sensor 150' is positioned in the coronary sinus when shunt device 100' is implanted in the tissue wall. In such examples, sensor 150' can be attached to one of proximal arms 132'. In further examples, an additional sensor can be included on shunt device 100' to position sensors in both the left atrium and the coronary sinus.

Sensor 150' is attached to shunt device 100' at sensor attachment portion 152'. Sensor 150' can be connected to sensor attachment portion 152' using any suitable attachment mechanism. For example, sensor 150' and sensor attachment portion 152' can include complimentary mating features. Sensor attachment portion 152' can be an extension of one of arms 114' of shunt device 100'. In some examples, sensor attachment portion 152' is an extension of distal arm 130A'. In other examples, sensor attachment portion 152' is an extension of distal arm 130B' or one of proximal arms 132'. Alternatively, as shown in FIG. 5, sensor attachment portion 152' can be a separate split arm portion of one of arms 114'. Sensor attachment portion 152' can be angled away from a horizontal reference plane (not shown) that is in the plane of the tissue wall adjacent to shunt device 100' when shunt device 100' is implanted in the tissue wall. That is, sensor attachment portion 152' can be angled away from the tissue wall.

Sensor 150' can be a pressure sensor to sense a pressure in the left atrium. In other examples, sensor 150' can be any sensor to measure a parameter in the left atrium. In yet other examples, sensor 150' can be any sensor to measure a parameter in the coronary sinus. Sensor 150' can include a transducer, control circuitry, and an antenna in one example. The transducer, for example a pressure transducer, is configured to sense a signal from the left atrium. The transducer can communicate the signal to the control circuitry. The control circuitry can process the signal from the transducer or communicate the signal from the transducer to a remote device outside of the body using the antenna. Sensor 150' can include alternate or additional components in other examples. Further, the components of sensor 150' can be held in a sensor housing that is hermetically sealed.

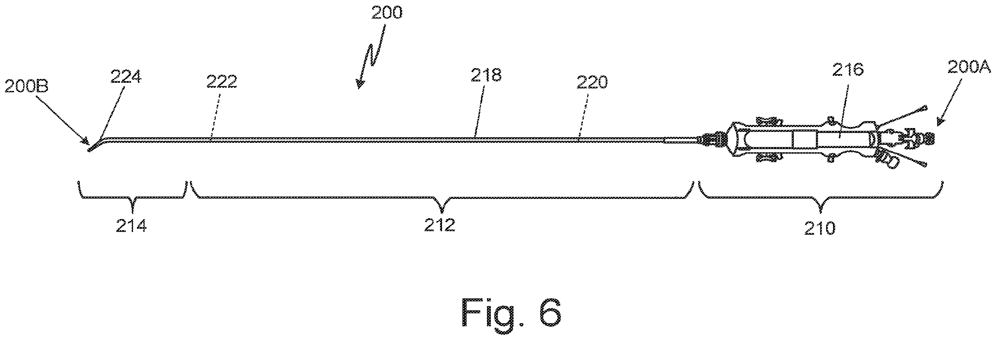

### DELIVERY CATHETER 200 (FIGS. 6-7B)

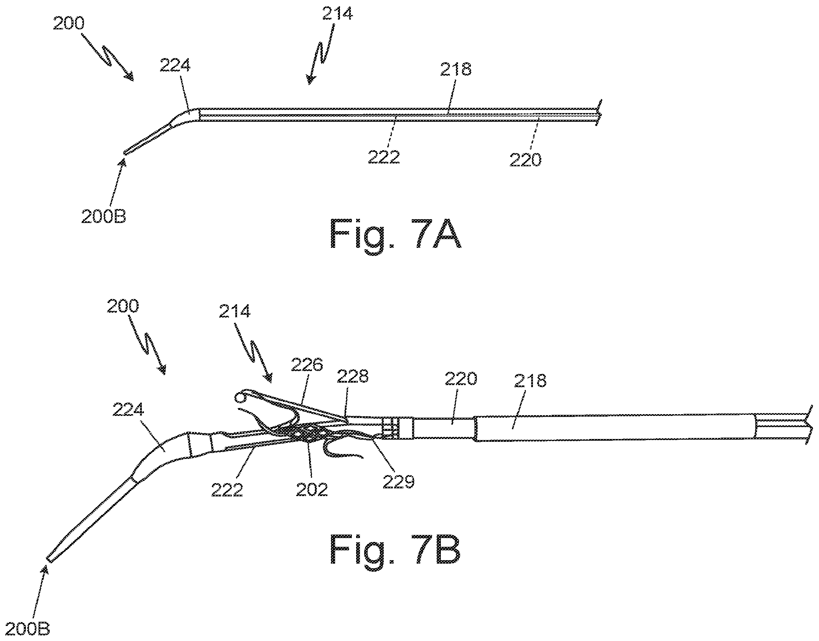

FIG. 6 is a side view of delivery catheter 200. FIG. 7A is a side view of distal portion 214 of delivery catheter 200 in a sheathed state. FIG. 7B is a side view of distal portion 214 of delivery catheter 200 in an unsheathed state. FIGS. 6, 7A, and 7B will be discussed together. FIGS. 6-7B show delivery catheter 200. FIG. 7B shows shunt device 202. Delivery catheter 200 includes proximal end 200A, distal end 200B, proximal portion 210, intermediate portion 212, distal portion 214, handle 216, outer sheath 218, inner sheath 220, bridge 222, nosecone 224, actuation rod 226, side opening 228, and notch 229.

Delivery catheter 200 is one example of a delivery catheter that can be used to implant a shunt device into a patient. Delivery catheter 200 as shown in FIGS. 6-7B is used to implant shunt device 202 (shown in FIG. 7B). Delivery catheter 200 can take other forms in alternate examples. Shunt device 202 can have the structure and design of any suitable shunt device, for example shunt device 100 or 100' as shown in FIGS. 3A-5. Delivery catheter 200 is shown as being configured to implant shunt device 202 without a sensor in the example shown in FIGS. 6-7B. In alternate examples, delivery catheter 200 can be used to implant a shunt device with a sensor, including any needed modifications to accommodate the sensor.

Delivery catheter 200 includes proximal portion 210 adjacent proximal end 200A of delivery catheter 200, intermediate portion 212 extending from proximal portion 210, and distal portion 214 extending from intermediate portion 212 to distal end 200B of delivery catheter 200. Proximal portion 210 includes handle 216, which can be grasped by a physician to control movement of delivery catheter 200. Handle 216 includes a number of ports through which guide wires, tubes, fluids, or other components or elements may be passed.

Intermediate portion 212 extends outward from handle 216 and is a length of catheter that can be moved through a patient. Outer sheath 218 and inner sheath 220 extend outward from handle 216 and form a portion of intermediate portion 212. Outer sheath 218 covers inner sheath 220.

Distal portion 214 extends from intermediate portion 212. Distal portion 214 includes bridge 222 and nosecone 224. Bridge 222 extends from inner sheath 220 towards nosecone 224. Nosecone 224 extends from bridge 222 to distal end 200B of delivery catheter 200. Bridge 222 is configured to hold shunt device 202. As shown in FIG. 7A, when delivery catheter 200 is in a sheathed state, outer sheath 218 will extend over and cover shunt device 202 on bridge 222. As shown in FIG. 7B, when delivery catheter 200 is in an unsheathed state, outer sheath 218 will be pulled back to expose bridge 222 and shunt device 202 on bridge 222. Nosecone 224 extends outward from bridge 222 and helps guide delivery catheter 200 through a patient's vasculature. Actuation rod 226, also called an actuation arm, extends through a lumen in inner sheath 220 and bridge 222. Actuation rod 226 emerges from side opening 228 in bridge 222 and connects to a first proximal arm of shunt device 202. Side opening 228 extends into a body of bridge 222. Notch 229 extends into the body of bridge 222 opposite side opening 228. Notch 229 is configured to seat a second proximal arm of shunt device 202. The second proximal arm can be retained on bridge 222 prior to deployment by a release wire (not shown) extending through a lumen of bridge 222 and through notch 229.

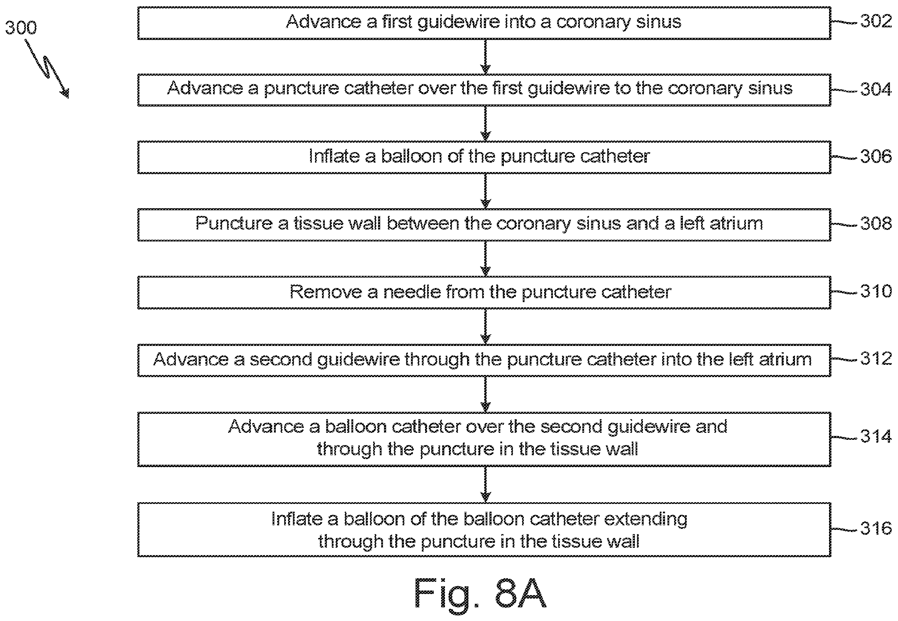

Delivery catheter 200 will be discussed below in more detail with respect to FIGS. 8A-9Q.

### DELIVERY METHOD 300 (FIGS. 8A-9Q)

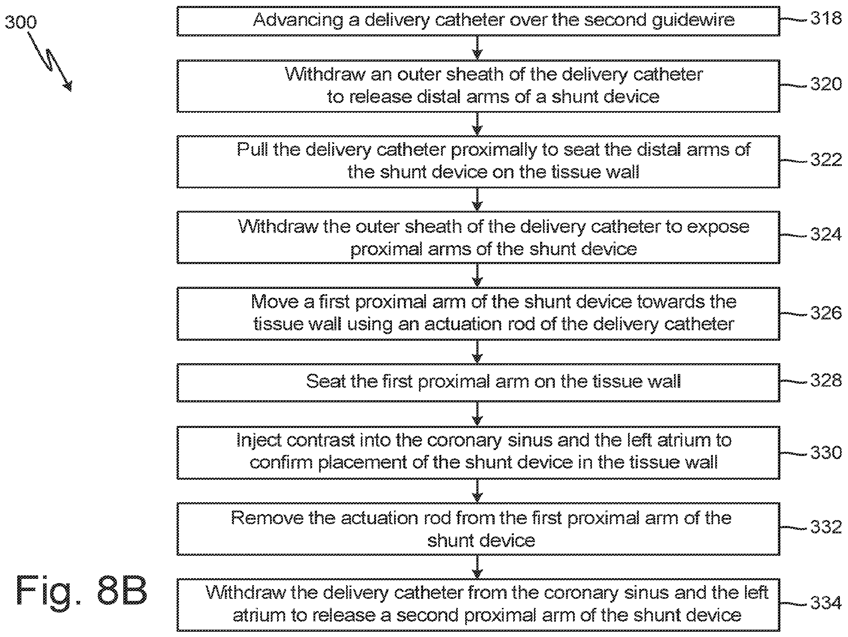

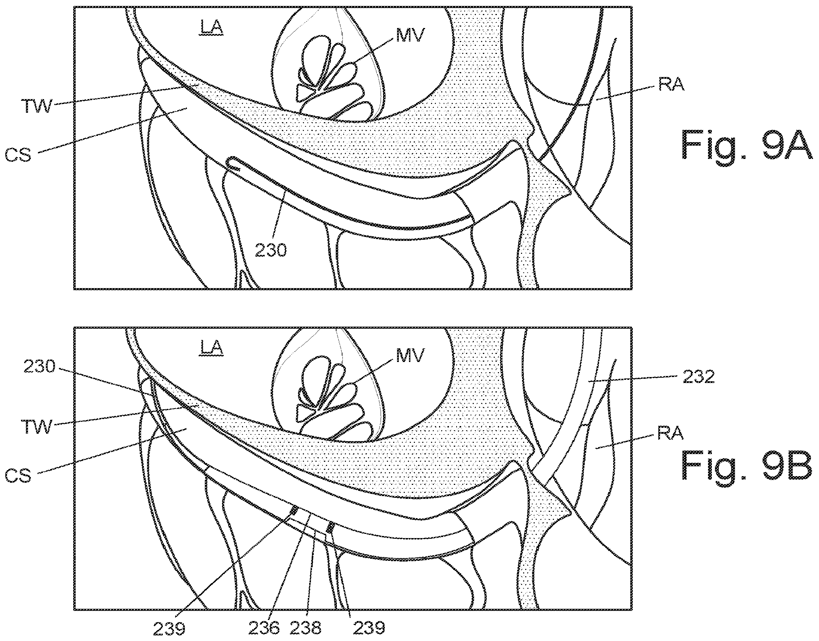

FIG. 8A is a flow chart showing steps for creating a puncture in tissue wall TW between coronary sinus CS and left atrium LA. FIG. 8B is a flow chart showing steps for implanting shunt device 202 in tissue wall TW between coronary sinus CS and left atrium LA. FIGS. 9A-9Q are schematic views showing the steps for implanting shunt device 202 in tissue wall TW between coronary sinus CS and left atrium LA. FIGS. 8A-9Q will be discussed together. FIGS. 8A-8B show method 300. FIG. 8A shows steps 302-316 of method 300. FIG. 8B shows steps 318-334 of method 300.

Step 302 includes advancing guidewire 230 into coronary sinus CS, as shown in FIG. 9A. Guidewire 230 can be inserted using traditional methods. Guidewire 230 is inserted into right atrium RA, through an ostium of coronary sinus CS, and then into coronary sinus CS. Optionally, a catheter having radiopaque markers can be inserted over guidewire 230 and imaging can be done to confirm placement of guidewire 230 in coronary sinus CS. Additionally, contrast can be injected into coronary sinus CS through the catheter to further confirm placement of guidewire 230 in coronary sinus CS. The catheter can then be removed once placement of guidewire 230 in coronary sinus CS is confirmed.

Step 304 includes advancing puncture catheter 232 over guidewire 230 to coronary sinus CS, as shown in FIG. 9B. Puncture catheter 232 is used to puncture tissue wall TW between coronary sinus CS and left atrium LA. Puncture catheter 232 includes catheter body 234 having opening 236 on a first side and balloon 238 on a second side opposite opening 236. Puncture catheter 232 can also include radiopaque markers 239 proximal and distal to opening 236 to confirm placement of puncture catheter 232 in coronary sinus CS. Puncture catheter 232 is advanced into coronary sinus CS so that opening 236 is facing tissue wall TW between coronary sinus CS and left atrium LA. Puncture catheter 232 shown in FIG. 9B is one example of a puncture catheter. In alternate examples, tissue wall TW can be punctured using other puncture catheters or other suitable mechanisms.

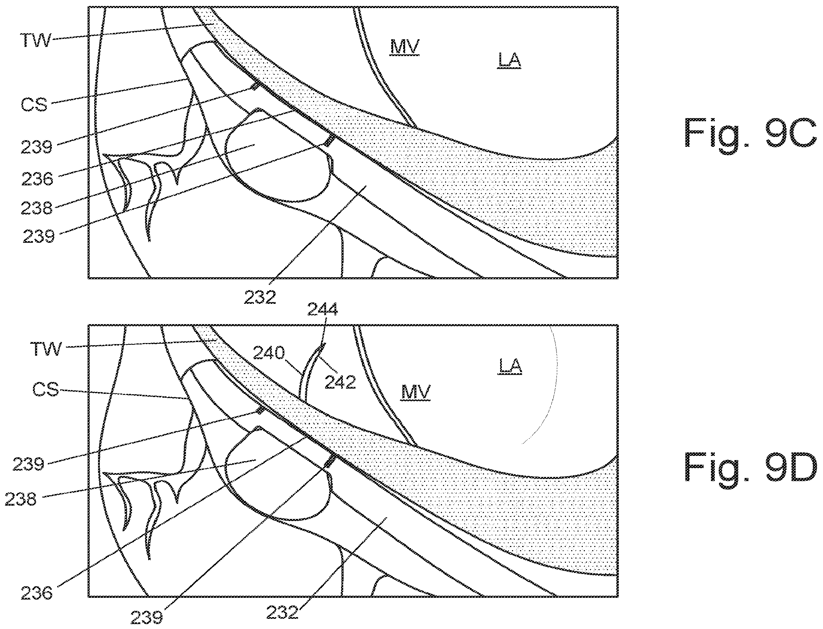

Step 306 includes inflating balloon 238 of puncture catheter 232, as shown in FIG. 9C. As balloon 238 is inflated, it will press against coronary sinus CS opposite of tissue wall TW. The inflation of balloon 238 will press puncture catheter 232 against tissue wall TW. Specifically, opening 236 will be pressed against tissue wall TW. Balloon 238 will anchor puncture catheter 232 in position in coronary sinus CS while a puncture is made in tissue wall TW. In alternate examples, any other suitable anchoring mechanism can be used instead of balloon 238. In further examples, step 306 is not needed.

Step 308 includes puncturing tissue wall TW between coronary sinus CS and left atrium LA, as shown in FIG. 9D. Puncture catheter 232 includes puncture arm 240 extending through a lumen in puncture catheter 232. Puncture arm 240 includes sheath 242 and needle 244 positioned in sheath 242 so that it extends out a distal end of puncture sheath 242. Puncture arm 240 can be advanced through puncture catheter 232 and out of opening 236 to puncture through tissue wall TW between coronary sinus CS and left atrium LA.

Puncture catheter 232 should be positioned in coronary sinus CS so that opening 236 of puncture catheter 232 is positioned 2-4 centimeters from the ostium of coronary sinus CS. This will position the puncture through tissue wall TW at the same location. The puncture, and ultimately the placement of shunt device 202 in the puncture, is positioned over the posterior leaflet of mitral valve MV.

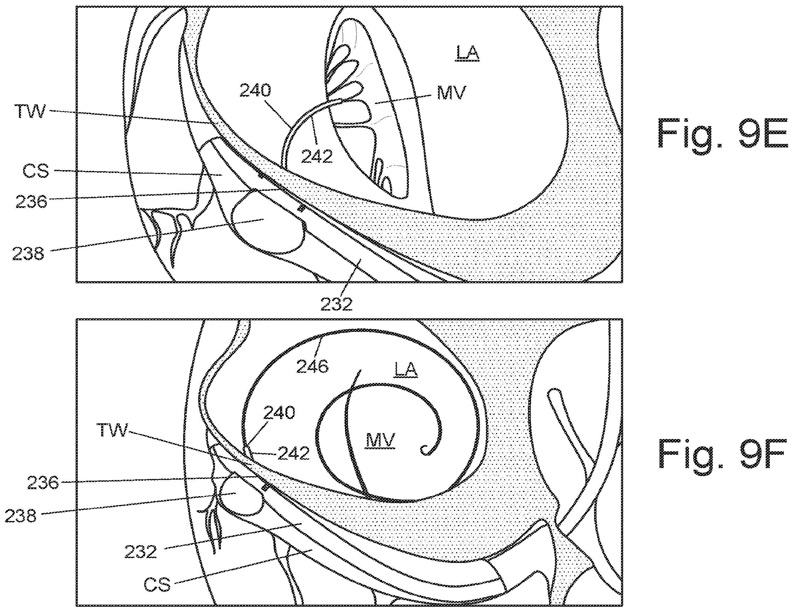

Step 310 includes removing needle 244 from puncture catheter 232, as shown in FIG. 9E. Needle 244 can be removed by pulling it proximally through a lumen extending through needle sheath 242 of puncture arm 240. Needle 244 is fully removed from puncture catheter 232, leaving a lumen extending from a proximal end of puncture catheter 232 through a distal end of needle sheath 242.

Step 312 includes advancing guidewire 246 through puncture catheter 232 into left atrium LA, as shown in FIG. 9F. Specifically, guidewire 246 is advanced through a lumen extending through a proximal end of puncture catheter 232 and needle sheath 242 of puncture arm 240. Guidewire 246 is advanced into left atrium LA until it coils in left atrium LA, as shown in FIG. 9F. Once guidewire 246 is fully positioned in left atrium LA, puncture catheter 232 and guidewire 230 can be removed from left atrium LA and coronary sinus CS.

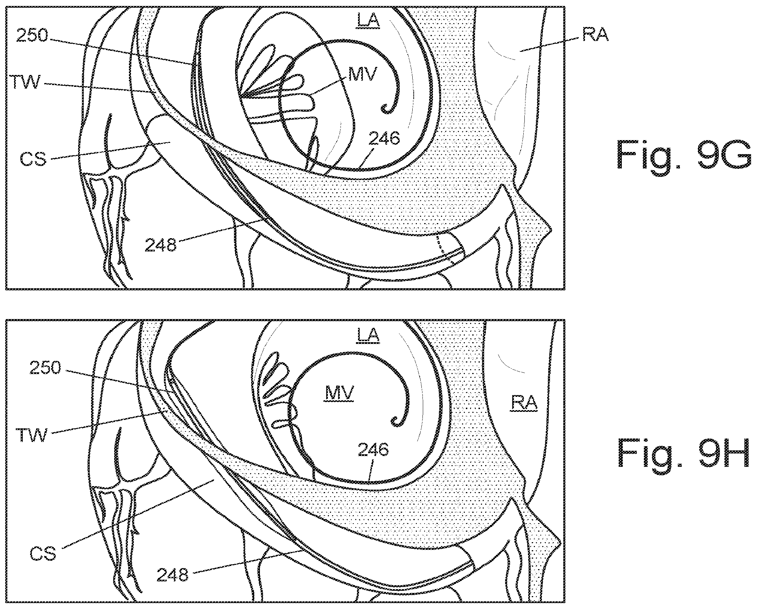

Step 314 includes advancing balloon catheter 248 over guidewire 246 and through the puncture in tissue wall TW, as shown in FIG. 9G. Balloon catheter 248 is advanced through the puncture in tissue wall TW so balloon 250 of balloon catheter 248 is positioned in the puncture in tissue wall TW. Balloon catheter 248 is shown as being a separate device from puncture catheter 232 in the example shown in FIG. 9G. However, in alternate examples, balloon catheter 248 can be inserted through puncture catheter 232 and through the puncture in tissue wall TW.

Step 316 includes inflating balloon 250 of balloon catheter 248 extending through the puncture in tissue wall TW, as shown in FIG. 9H. Balloon 250 extends along a distal portion of balloon catheter 248. As balloon 250 is inflated, it will expand and push open the tissue surrounding the puncture in tissue wall TW. The inflation of balloon 250 will cause the puncture in tissue wall TW to become a wider opening in which a shunt device can be positioned. Balloon 250 can then be deflated and balloon catheter 248 can be removed from left atrium LA and coronary sinus CS.

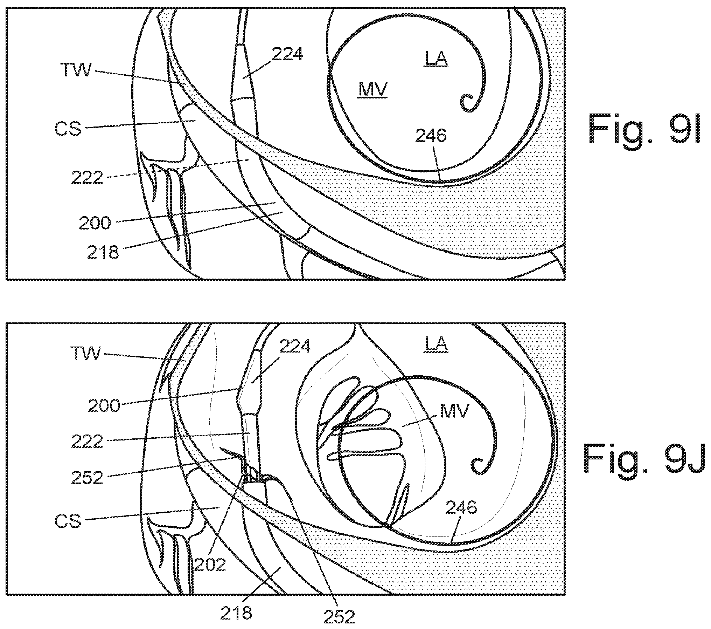

Step 318 includes advancing delivery catheter 200 over guidewire 246, as shown in FIG. 9I. Delivery catheter 200 has the general structure and design as discussed with reference to FIGS. 6-7B above. Delivery catheter 200 is inserted through coronary sinus CS, through the opening in tissue wall TW, and into left atrium LA. When delivery catheter 200 is properly positioned in tissue wall TW, nosecone 224 will be positioned in left atrium LA, and bridge 222 will extend through tissue wall TW between left atrium LA and coronary sinus CS. Nosecone 224 tapers from a smaller diameter at a distal end to a larger diameter at a proximal end. The taper of nosecone 224 helps to advance nosecone 224 through the opening in tissue wall TW and widens the opening as needed. Bridge 222 holds shunt device 202 (not shown in FIG. 9I) in a collapsed position on bridge 222. Bridge 222 is positioned in tissue wall TW so that shunt device 202 is generally positioned in the opening in tissue wall TW for deployment into the opening.

Step 320 includes withdrawing outer sheath 218 of delivery catheter 200 to release distal arms 252 of shunt device 202, as shown in FIG. 9J. Outer sheath 218 can be withdrawn to expose part of shunt device 202 held on bridge 222 of delivery catheter 200. As outer sheath 218 is withdrawn, distal arms 252 of shunt device 202 will be released and assume their preset shape. Delivery catheter 200 should be positioned in left atrium LA such that when outer sheath 218 is withdrawn to release distal arms 252 of shunts device 202, distal arms 252 of shunt device 202 are positioned in left atrium LA.

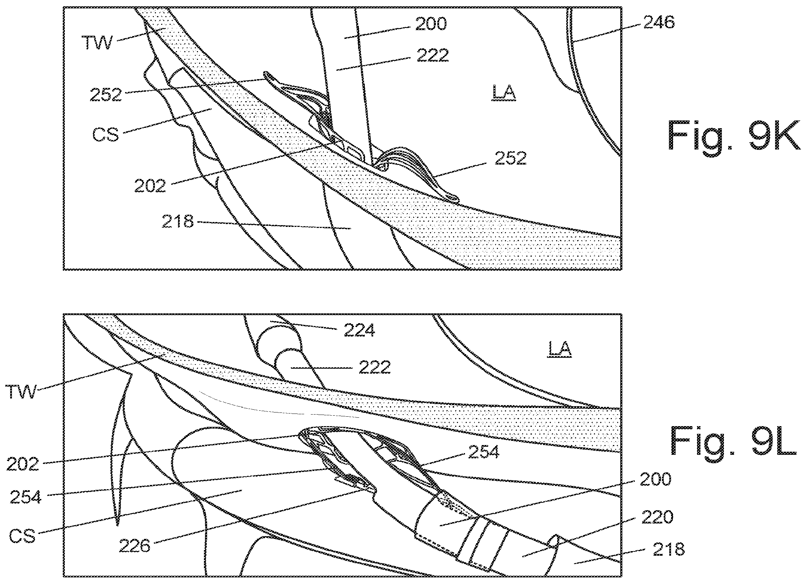

Step 322 includes pulling delivery catheter 200 proximally to seat distal arms 252 of shunt device 202 on tissue wall TW, as shown in FIG. 9K. Delivery catheter 200 can be gently pulled proximally to seat distal arms 252 of shunt device 202 on tissue wall TW in left atrium LA. A physician should stop gently pulling on delivery catheter 200 when resistance is sensed, indicating that distal arms 252 have come into contact with tissue wall TW. This will also position a central flow tube of shunt device 202 in the opening in tissue wall TW.

Step 324 includes withdrawing outer sheath 218 of delivery catheter 200 to expose proximal arms 254 of shunt device 202, as shown in FIG. 9L. Outer sheath 218 is withdrawn a set distance to fully expose shunt device 202, including proximal arms 254 of shunt device 202. Delivery catheter 200 should be positioned in left atrium LA, tissue wall TW, and coronary sinus CS so that proximal arms 254 will be positioned in coronary sinus CS when outer sheath 218 is withdrawn. Proximal arms 254 are constrained on bridge 222 of delivery catheter 200 and will not automatically assume their preset shape when outer sheath 218 is withdrawn.

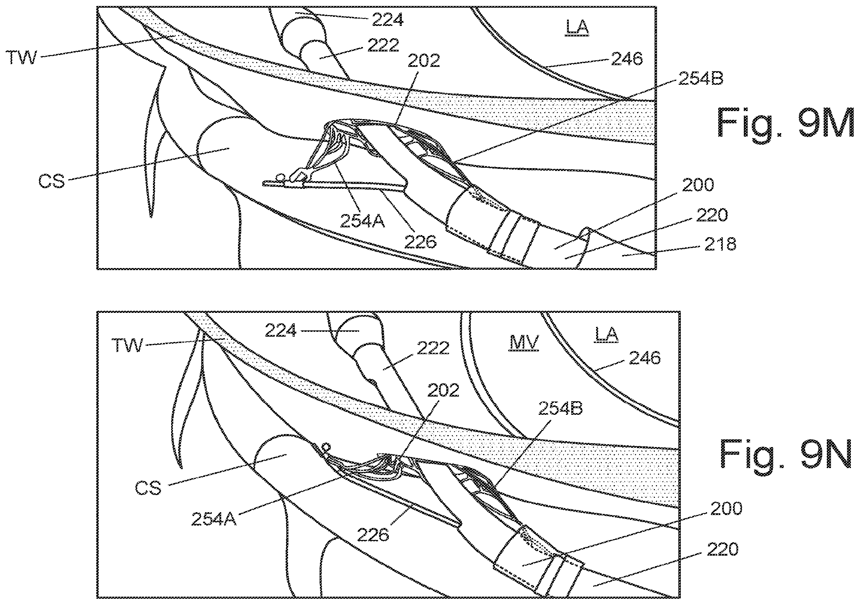

Step 326 includes moving first proximal arm 254A of shunt device 202 towards tissue wall TW using actuation rod 226 of delivery catheter 200, as shown in FIG. 9M. Actuation rod 226 extends through a lumen in delivery catheter 200 and can be actuated forward to move first proximal arm 254A towards tissue wall TW.

Step 328 includes seating first proximal arm 254A on tissue wall TW, as shown in FIG. 9N. Actuation rod 226 of delivery catheter 200 is actuated fully outward to seat first proximal arm 254A on tissue wall TW. When first proximal arm 256A is seated on tissue wall TW, it will be positioned in coronary sinus CS.

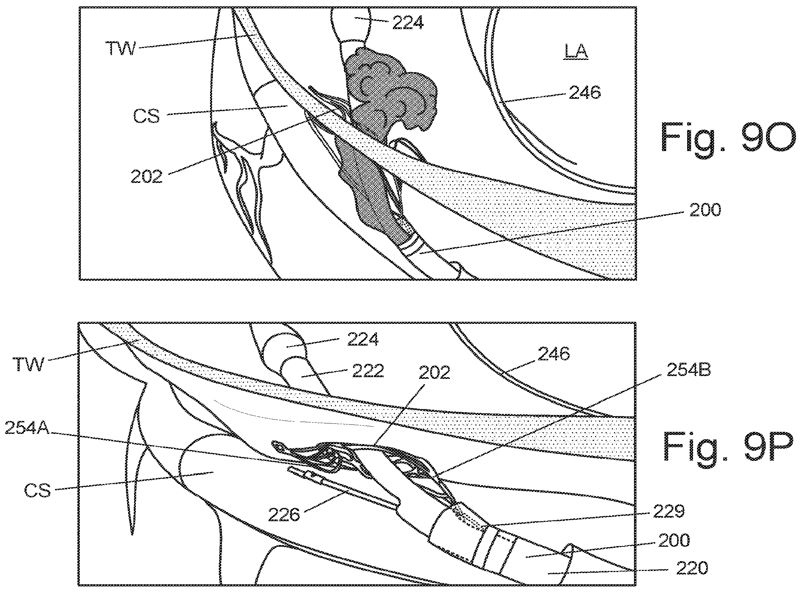

Step 330 includes confirming placement of shunt device 202 in tissue wall TW. FIG. 9O illustrates a known method for confirming tissue confirmation, which includes injecting a contrast agent through a lumen extending through delivery catheter 200. The contrast agent can move through coronary sinus CS and left atrium LA. The contrast will highlight shunt device 202 under fluoroscopy to confirm proper placement of distal arms 252 and first proximal arm 254A of shunt device 202 on tissue wall TW. Improved methods of confirming tissue capture are discussed below.

Step 332 includes removing actuation rod 226 from first proximal arm 254A of shunt device 202, as shown in FIG. 9P. Actuation rod 226 can be held on and removed from first proximal arm 254A using any suitable mechanism. In the example shown in FIG. 9P, a release wire holds actuation rod 226 on first proximal arm 254A. The release wire can be withdrawn proximally to disconnect release wire from first proximal arm 254A. Actuation rod 226 can then be pulled proximally through a lumen of delivery catheter 200 to remove actuation rod 226 from coronary sinus CS.

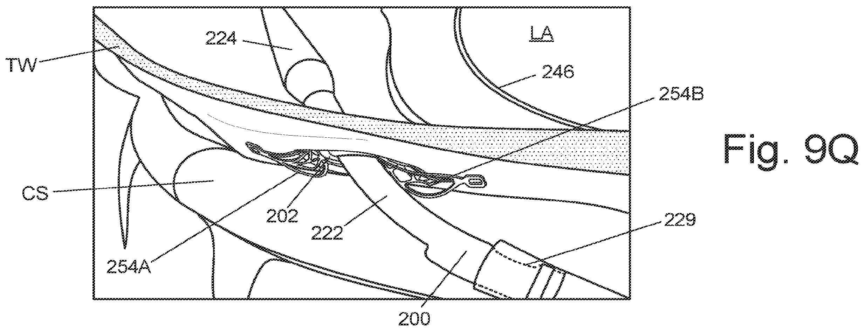

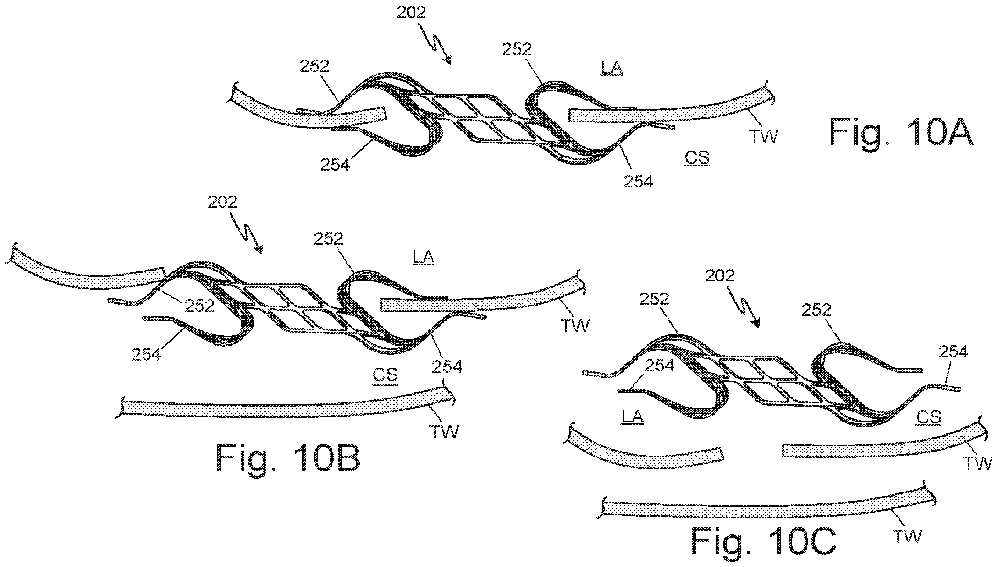

Step 334 includes withdrawing delivery catheter 200 from coronary sinus CS and left atrium LA to release second proximal arm 254B of shunt device 202, as shown in FIG. 9Q. Second proximal arm 254B is held in place on bridge 222 in notch 229 formed in bridge 222. As delivery catheter 200 is withdrawn, second proximal arm 254B will be released from notch 229 in bridge 222 and take its preset shape. Specifically, second proximal arm 254B will seat upon tissue wall TW as it takes its preset shape. Second proximal arm 245B will be positioned in coronary sinus CS. After second proximal arm 254B is seated on tissue wall TW, shunt device 202 will be fully deployed in tissue wall TW, as shown in FIG. 10A. Delivery catheter 200 and guidewire 246 can then be removed from left atrium LA and coronary sinus CS.

Method 300 is one example of a method that can be used to implant shunt device 202 in tissue wall TW between left atrium LA and coronary sinus CS. Method 300 can include fewer, more, or different steps in alternate examples. Further, puncture catheter 232 and delivery catheter 200 are shown as being separate catheters in the example shown in FIGS. 9A-9Q but can be a single catheter in alternate examples.

Shunt devices must be anchored in place to avoid displacement during normal heart rhythms. Techniques are needed to confirmed proper placement of shunt devices during implantation.

FIG. 10A is a simplified perspective view of shunt device 202 properly seated between left atrium LA and coronary sinus CS. FIG. 10B is a simplified perspective view of shunt device 202 improperly seated between left atrium LA and coronary sinus CS. FIG. 10C is a simplified perspective view of shunt device 202 embolized in left atrium LA. FIGS. 10A-10B will be discussed together. FIGS. 10A-10B show shunt device 202, including distal arms 252 and proximal arms 254. FIGS. 10A-10B further show left atrium LA, coronary sinus CS, and tissue wall TW.

FIG. 10A shows shunt device 202 properly seated in tissue wall TW between left atrium LA and coronary sinus CS. As illustrated, distal arms 252 engage tissue wall TW and are positioned in left atrium LA and proximal arms 254 engage tissue wall TW and are positioned in coronary sinus CS. During deployment, one or more distal arms 252 or proximal arms 254 can be mis-seated. In one example, shunt device 202 could be improperly seated such that one or more of distal arms 252 is positioned in coronary sinus CS rather than in left atrium LA, as illustrated in FIG. 10B. For example, during an implantation procedure (e.g., during step 322 of method 300 as shown in FIG. 8B), a physician may pull delivery catheter 200 back too hard after distal arms 252 are released, causing all or a part of shunt device 202 to be pulled into coronary sinus CS. In another example, shunt device 202 could be improperly seated such that the entirety of shunt device 202 is located in left atrium LA, as illustrated in FIG. 10C. For example, during an implantation procedure (e.g., during step 322 of method 300 as shown in FIG. 8B), a physician may not pull delivery catheter 200 back far enough after distal arms 252 are released, so one or more of proximal arms 254 may be released in or pushed through to left atrium LA, causing shunt device 202 to embolize. Confirming tissue capture between the arms of a shunt device helps a physician to determine when it is safe to release the shunt device. As such, confirming proper seating of the shunt device during and/or following delivery also helps reduce the risk of embolization and/or need for redeployment. The present disclosure includes several contrast delivery devices for confirming tissue capture and ensuring proper placement of a shunt device.

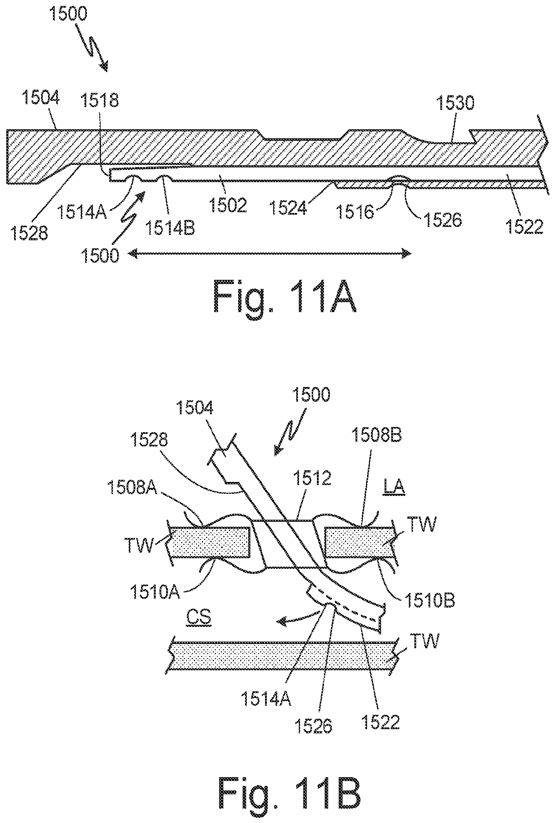

### DELIVERY DEVICE 1500 (FIGS. 11A-14)

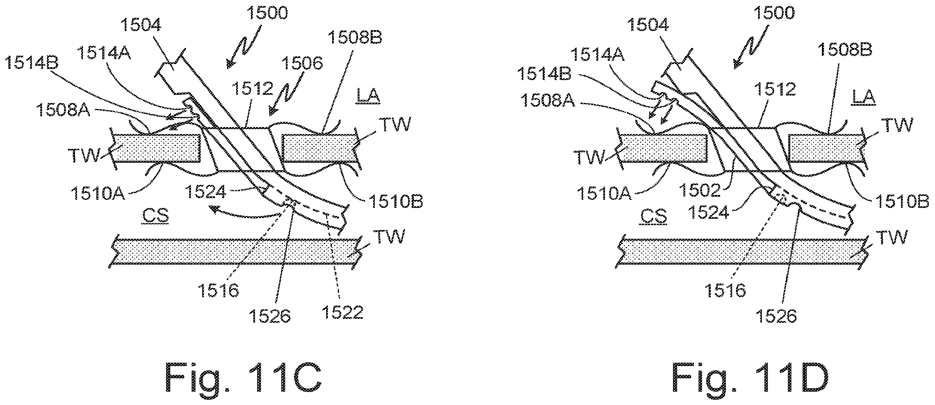

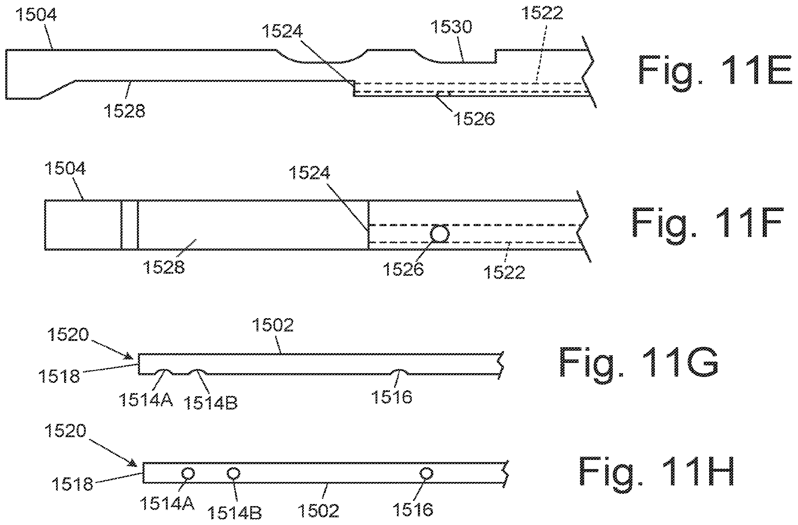

FIG. 11A is a cross-sectional view of delivery device 1500 for delivering and placing a shunt device within a human body and confirming tissue capture of the shunt device with contrast delivery via adjustable contrast tube 1502. FIG. 11B is a simplified perspective view of delivery device 1500 of FIG. 11A with adjustable contrast tube 1502 in a first position. FIG. 11C is a simplified perspective view of delivery device 1500 of FIG. 11A with adjustable contrast tube 1502 in a second position. FIG. 11D is a simplified perspective view of delivery device 1500 of FIG. 11A with adjustable contrast tube 1502 in a third position. FIG. 11E is side view of bridge 1504 of delivery device 1500. FIG. 11F is a bottom view of bridge 1504. FIG. 11G is a side view of adjustable contrast tube 1502. FIG. 11H is a bottom view of adjustable contrast tube 1502. FIGS. 11A-11H will be discussed together. Delivery device 1500, adjustable contrast tube 1502, bridge 1504, shunt device 1506, distal arms 1508A and 1508B, proximal arms 1510A and 1510B, central flow tube 1512, left atrium LA, coronary sinus CS, tissue wall TW, distal holes 1514A and 1514B, proximal hole 1516, distal terminal end 1518, distal tip 1520, lumen 1522, distal end 1524, exit hole 1526, side opening 1528, and notch 1530 are shown.

Delivery device 1500 is one example of delivery catheter 200 shown in FIG. 7B to deliver shunt device 1506 and confirm placement of shunt device 1506 in tissue wall TW. Shunt device 1506 can be deployed as described in method 300 shown in FIGS. 8A, 8B, and 9A-9Q and described with respect thereto with a modification of the step of contrast delivery for tissue capture confirmation shown in FIG. 9O.

Shunt device 1506 can be substantially the same as or similar to shunt devices 100 and 100' illustrated in FIGS. 3A, 3B, 4, and 5 and described with respect thereto. Shunt device 1506 includes distal arms 1508A and 1508B, proximal arms 1510A and 1510B, and central flow tube 1512.

Delivery device 1500 includes bridge 1504. Bridge 1504 can be substantially similar to bridge 222 of delivery catheter 200 shown in FIG. 7B with the addition of exit hole 1526. Bridge 1504 includes lumen 1522 with distal end 1524, exit hole 1526, notch 1530, and side opening 1528. Distal end 1524 of lumen 1522 opens to side opening 1528. In the orientation shown in FIGS. 11E and 11F, side opening 1528 is positioned on a bottom side of bridge 1504 opposite notch 1530. Notch 1530 is positioned proximal to side opening 1528. Exit hole 1526 is positioned on the bottom side of bridge 1504 proximal to side opening 1528. Exit hole 1526 is open to a side of lumen 1522.

Notch 1530 can be substantially the same as notch 229 of bridge 222 as described with respect to FIG. 7B. Notch 1530 cuts into the body of bridge 1504 opposite side opening 1528. Notch 1530 can be configured to seat proximal arm 1510B of shunt device 1506.

Side opening 1528 can be substantially the same as side opening 228 as described with respect to FIG. 7B. Side opening 1528 cuts into the body of bridge 1504 and extends lengthwise along the side of bridge 1504. Side opening 1528 is positioned adjacent to distal end 1524 of lumen 1522.

Lumen 1522 extends lengthwise through at least a portion of bridge 1504. Lumen 1522 can be one of a plurality of lumens disposed in bridge 1504 as previously described with respect to bridge 222 shown in FIG. 7B. Lumen 1522 is a hollow shaft having distal end 1524. Distal end 1524 opens to side opening 1528. Distal end 1524 can be positioned at or substantially at an innermost extent of side opening 1528. For example, as illustrated in FIGS. 11A and 11E, side opening 1528 can be cut into the body of bridge 1504 such that an innermost extent of side opening 1528 meets an inner diameter of lumen 1522 at distal end 1524. As illustrated in FIG. 11F, lumen 1522 can be positioned off-center if necessary to accommodate one or more additional lumens. For example, lumen 1522 can be positioned to accommodate a lumen for actuation rod 226 as shown in FIG. 7B, which also extends into side opening 1528 as described with respect to delivery catheter 200 in FIGS. 6-7B. Lumen 1522 is configured to receive adjustable contrast tube 1502. Lumen 1522 has an inner diameter sized to accommodate a sliding motion of adjustable contrast tube 1502 within lumen 1522 to allow a user to advance and retract adjustable contrast tube 1502 through lumen 1522 during contrast delivery procedures to contain a contrast agent in contrast tube 1502 while preventing leakage of the contrast agent between an inner diameter wall of lumen 1522 and outer diameter wall of adjustable contrast tube 1502. In some examples, lumen 1522 can have an inner diameter approximately 0.01 in. (0.254 mm) larger than an outer diameter of adjustable contrast tube 1502. Adjustable contrast tube 1502 can have a minimum outer diameter of approximately 0.042 in. (1.07 mm) to promote the flow of the contrast agent during injection without requiring application of excessive force by the user and without generating excessive exit pressure, to protect tissue wall TW.

Exit hole 1526 is positioned on a side of bridge 1504. Exit hole 1526 is aligned with lumen 1522 and connects lumen 1522 to an exterior surface of bridge 1504. Exit hole 1526 is located proximal to side opening 1528 on bridge 1504. As described further herein, exit hole 1526 is configured to deliver a contrast agent received from adjustable contrast tube 1502 to a targeted area of interest (e.g., to coronary sinus CS to confirm tissue capture of shunt device 1506). It will be understood by one of ordinary skill in the art that exit hole 1526 can be positioned at other locations on bridge 1504 to provide targeted delivery of the contrast agent and is not limited to the location illustrated.

Adjustable contrast tube 1502 is configured to deliver a contrast agent (e.g., contrast dye or media visible via medical imaging). Adjustable contrast tube 1502 includes distal tip 1520, distal terminal end 1518, at least one distal hole 1514A, 1514B, and proximal hole 1516. Distal tip 1520 is located at a distal end of adjustable contrast tube 1502 and terminates at distal terminal end 1518. Distal tip 1520 includes one or more distal holes 1514A and 1514B. The distal end is located opposite a proximal end (not shown) of adjustable contrast tube 1502. The proximal end of adjustable contrast tube 1502 can be coupled to a contrast agent mechanism. The contrast agent is injected into adjustable contrast tube 1502 at the proximal end of adjustable contrast tube 1502. Proximal hole 1516 and distal holes 1514A and 1514B are spaced along a length of adjustable contrast tube 1502 with distal holes 1514A and 1514B disposed closer to distal terminal end 1518 than proximal hole 1516. Proximal hole 1516 and distal holes 1514A and 1514B are configured to deliver the contrast agent to targeted regions in one or both of left atrium LA and coronary sinus CS and around shunt device 1506. As illustrated in FIGS. 11A-11D, 11G, and 11H and described herein, distal tip includes two distal holes 1514A and 1514B. Distal hole 1514B is disposed between proximal hole 1516 and distal hole 1514A. In alternative examples, distal tip can include a single distal hole or more distal holes than the two distal holes illustrated as needed for targeted delivery of the contrast agent. It will be understood by one of ordinary skill in the art that the number of distal holes can be modified as needed to provide targeted contrast delivery in varying applications. Spacing between proximal hole 1516 and distal hole 1514A and/or 1514B can be configured to direct the contrast agent to separate target regions, such as coronary sinus CS and left atrium LA as illustrated by arrows depicting flow of the contrast agent in FIGS. 11B-D. Spacing between distal holes 1514A and 1514B can be designed to direct the contrast agent to a single region and/or targeted locations in a single region, such as toward distal arm 1508A as illustrated by arrows depicting flow of the contrast agent in FIG. 11D. In some examples, proximal hole 1516 can be spaced from distal hole 1514B by approximately 1 in. (25.4 mm) and distal hole 1514B can be spaced from distal hole 1514A by approximately 0.1 in. (2.54 mm). It will be understood by one of ordinary skill in the art that the spacing between proximal hole 1516, distal hole 1514A, and distal hole 1514B can be modified as needed to provide targeted contrast delivery in varying applications. Distal terminal end 1518 is closed. As such, all contrast agent is delivered from adjustable contrast tube 1502 via proximal hole 1516 and distal holes 1514A and 1514B.

As illustrated in FIGS. 11B-11D, adjustable contrast tube 1502 is slidably received in lumen 1522 and traversable between a plurality of positions to deliver the contrast agent, for example, toward distal arm 1508A and proximal arm 1510A. Adjustable contrast tube 1502 can be positioned in bridge 1504 in assembly of delivery device 1500 and advanced and/or retracted to provide targeted delivery of the contrast agent during deployment of shunt device 1506. FIG. 11B illustrates adjustable contrast tube 1502 in a first position; FIG. 11C illustrates adjustable contrast tube 1502 in a second position; FIG. 11D illustrates adjustable contrast tube 1502 in a third position. The position of adjustable contrast tube 1502 is not limited to the positions illustrated. The use of the terms "first," "second," and "third" does not limit the order in which adjustable contrast tube 1502 is positioned for delivery of the contrast agent.

A user can position adjustable contrast tube 1502 in each of the first, second, and third positions by advancing and/or retracting adjustable contrast tube 1502 from the proximal end of adjustable contrast tube 1502. A positioning mechanism (not shown) can be included, for example, on a proximal handle of the delivery catheter (e.g., handle 216 of FIG. 6) to allow a user to manipulate adjustable contrast tube 1502 and locate adjustable contrast tube 1502 at each of the first, second, and third positions. For example, a user can advance or retract adjustable contrast tube 1502 between pre-defined markings indicating the location of each of the first, second, and third positions of adjustable contrast tube 1502. As described further herein, adjustable contrast tube 1502 can be positioned at each of the first, second, and third positions to deliver the contrast agent to targeted regions. Adjustable contrast tube 1502 can be positioned in one of the first, second, or third positions upon assembly such that adjustable contrast tube 1502 is in position for delivery of the contrast agent upon delivery of shunt device 1506. The user can slide adjustable contrast tube 1502 to the next of the desired first, second, and third positions to deliver the contrast agent to another targeted region. The user can again slide adjustable contrast tube 1502 to the last of the desired first, second, and third positions to deliver the contrast agent to yet another targeted region. The contrast agent can be delivered from adjustable contrast tube 1502 from each of the first, second, and third positions any number of times to confirm proper placement or tissue capture of shunt device 1506.

Proximal hole 1516 and distal holes 1514A and 1514B can be aligned along the length of adjustable contrast tube 1502 such that each of proximal hole 1516 and distal holes 1514A and 1514B can be aligned with exit hole 1526 by advancing and retracting adjustable contrast tube 1502 in lumen 1522 without twisting adjustable contrast tube 1502. Each of proximal hole 1516 and distal holes 1514A and 1514B can have a diameter substantially matching a diameter of exit hole 1526. The diameter of proximal hole, distal holes 1514A and 1514B, and exit hole 1526 can be, for example, approximately 0.03 in. (0.762 mm) to promote the flow of the contrast agent during injection without requiring application of excessive force by the user.

As illustrated in FIG. 11B, distal hole 1514A is aligned with exit hole 1526 of bridge 1504 when adjustable contrast tube 1502 is in the first position. Distal hole 1514A is open to exit hole 1526 and thereby the outer surface of bridge 1504 to allow flow of the contrast agent when adjustable contrast tube 1502 is in the first position. Proximal hole 1516 and distal hole 1514B are located in lumen 1522 and abut a wall of lumen 1522 when adjustable contrast tube 1502 is in the first position and are effectively closed. Although there is a small gap between the outer diameter of adjustable contrast tube 1502 and the inner diameter of lumen 1522, the gap is generally too small for the contrast agent to leak from proximal hole 1516 and distal hole 1514B when contrast agent is injected into adjustable contrast tube 1502. The contrast agent is thereby preferentially delivered through distal hole 1514A and exit hole 1526 when adjustable contrast tube 1502 is in the first position. As illustrated in FIG. 11B, upon delivery of shunt device 1506, exit hole 1526 is positioned in the coronary sinus CS and positioned to deliver contrast agent toward proximal arm 1510A of shunt device 1506 when adjustable contrast tube 1502 is in the first position. In the first position, no openings from adjustable contrast tube 1502 are positioned in the left atrium and all injected contrast agent is preferentially delivered to coronary sinus CS when adjustable contrast tube 1502 is in the first position.

As illustrated in FIG. 11C, distal holes 1514A and 1514B are positioned in side opening 1528 and open to allow flow of the contrast agent when adjustable contrast tube 1502 is in the second position. Proximal hole 1516 is aligned with exit hole 1526 and open to allow flow of the contrast agent when adjustable contrast tube 1502 is in the second position. As illustrated in FIG. 11A, exit hole 1526 is positioned in the coronary sinus CS and side opening 1528 is positioned in the left atrium LA following deployment of shunt device 1506. Proximal hole 1516 is positioned to direct the contrast agent into the coronary sinus CS and toward proximal arm 1510A of shunt device 1506 when adjustable contrast tube 1502 is in the second position. Distal holes 1514A and 1514B are positioned to direct the contrast agent into the left atrium LA and toward distal arm 1508A of shunt device 1506 when adjustable contrast tube 1502 is in the second position. In the second position, the injected contrast agent is delivered to both coronary sinus CS and left atrium LA when adjustable contrast tube 1502 is in the second position.

As illustrated in FIG. 11D, distal holes 1514A and 1514B are positioned in side opening 1528 and open to allow flow of the contrast agent when adjustable contrast tube 1502 is in the third position. Proximal hole 1516 is positioned in lumen 1522 and abuts a wall of lumen 1522 such that proximal hole 1516 is effectively closed to flow of the contrast agent when adjustable contrast tube 1502 is in the third position. As illustrated in FIG. 11D, adjustable contrast tube 1502 is advanced between the second and third positions such that proximal hole 1516 is positioned distal to exit hole 1526. Alternatively, or in addition to the position shown in FIG. 11D, adjustable contrast tube 1502 can be retracted such that proximal hole 1516 is positioned in lumen 1522 proximal to exit hole 1526. As illustrated in FIG. 11A, side opening 1528 is positioned in the left atrium LA following deployment of shunt device 1506. Distal holes 1514A and 1514B are positioned to direct the contrast agent into the left atrium LA and toward distal arm 1508A of shunt device 1506 when adjustable contrast tube 1502 is in the third position. In the third position, no openings in adjustable contrast tube 1502 are positioned in coronary sinus CS and all injected contrast agent is preferentially delivered to left atrium LA when adjustable contrast tube 1502 is in the third position.

Adjustable contrast tube 1502 can be formed, for example, of a nitinol hypotube, polyamide braid or other materials known in the art, including combinations thereof, suitable for delivering a contrast agent. Adjustable contrast tube 1502 can be manufactured with a closed distal terminal end 1518. In alternative examples, distal tip 1520 can include a sleeve (not shown) formed of a flexible plastic and configured to close distal terminal end 1518 of adjustable contrast tube 1502. The sleeve can include holes sized to match distal holes 1514A and 1514B and positioned to align with distal holes 1514A and 1514B.

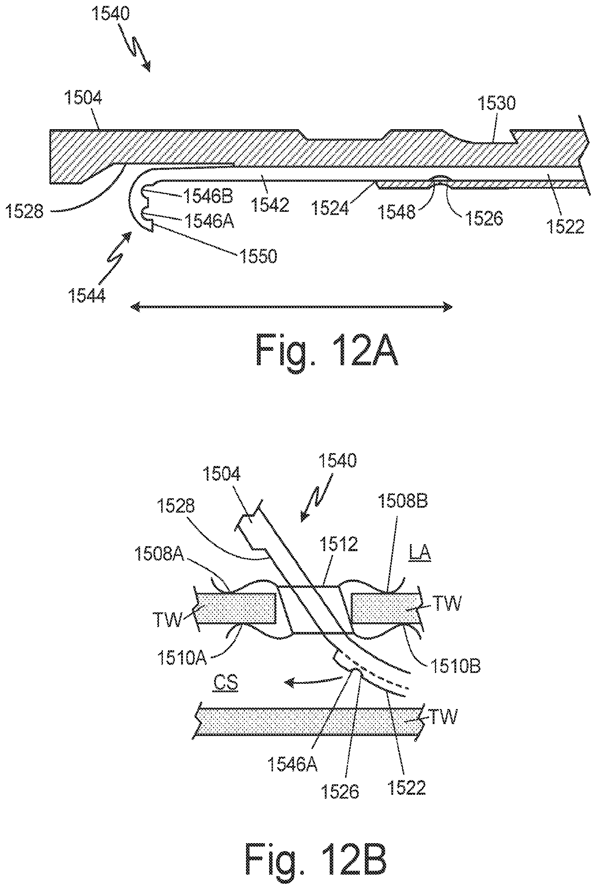

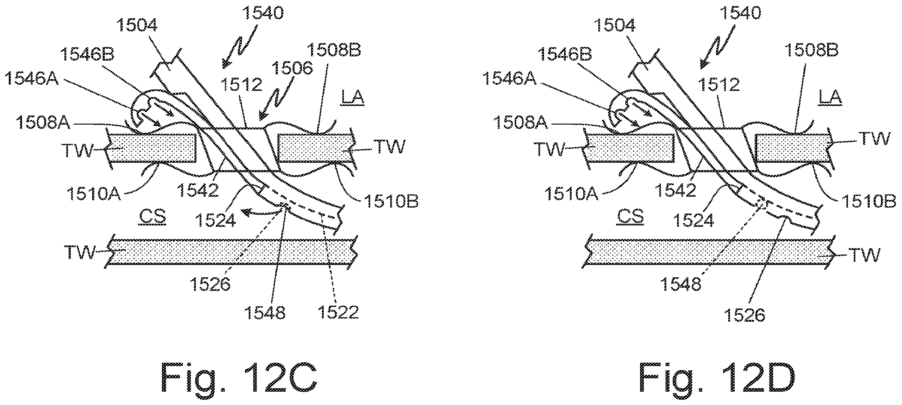

FIG. 12A is a cross-sectional view of delivery device 1540 for delivering and placing shunt device 1506 within a human body and confirming tissue capture of shunt device 1506 with contrast delivery via adjustable contrast tube 1542. FIG. 12B is a simplified perspective view of delivery device 1540 of FIG. 12A with adjustable contrast tube 1542 in a first position during contrast delivery. FIG. 12C is a simplified perspective view of delivery device 1540 of FIG. 12A with adjustable contrast tube 1542 in a second position during contrast delivery. FIG. 12D is a simplified perspective view of delivery device 1540 of FIG. 12A with adjustable contrast tube 1542 in a third position during contrast delivery. FIGS. 12A-12D will be discussed together. Delivery device 1540, shunt device 1506, distal arms 1508A and 1508B, proximal arms 1510A and 1510B, central flow tube 1512, tissue wall TW, adjustable contrast tube 1542, distal holes 1546A and 1546B, proximal hole 1548, distal terminal end 1550, distal tip 1544, bridge 1504, lumen 1522, distal end 1524, exit hole 1526, side opening 1528, and notch 1530 are shown.

Delivery device 1540 is substantially the same as delivery device 1500 illustrated in FIGS. 11A-11F and discussed with respect thereto, with the replacement of adjustable contrast tube 1502 with adjustable contrast tube 1542. Bridge 1504, including lumen 1522 with distal end 1524, exit hole 1526, and side opening 1528 is the same as illustrated in FIGS. 11A-11F and discussed with respect thereto. Shunt device 1506 is also the same as shunt device 1506 illustrated in FIGS. 11B-11D and discussed with respect thereto.

Adjustable contrast tube 1542 is substantially similar to adjustable contrast tube 1502 but includes curved distal tip 1544 configured to more closely direct the contrast agent to a targeted region of interest (e.g., distal arm 1508A of shunt device 1506 or a tissue wall TW of the left atrium LA). Adjustable contrast tube 1542 includes distal tip 1544, distal terminal end 1550, at least one distal hole 1546A, 1546B, and proximal hole 1548. Distal tip 1544 is located at a distal end of adjustable contrast tube 1542 and terminates at distal terminal end 1550. Distal tip 1544 includes one or more distal holes 1546A and 1546B. Proximal hole 1548 and distal holes 1546A and 1546B are spaced along a length of adjustable contrast tube 1542 with distal holes 1546A and 1546B disposed closer to distal terminal end 1550 than proximal hole 1548. As illustrated in FIGS. 12A-12D and described herein, distal tip 1544 includes two distal holes 1546A and 1546B. Distal hole 1546B is disposed between proximal hole 1548 and distal hole 1546A. In alternative examples, distal tip can include a single distal hole or more distal holes than the two distal holes 1546A and 1546B illustrated as needed for targeted delivery of the contrast agent. It will be understood by one of ordinary skill in the art that the number distal holes can be modified as needed to provide targeted contrast delivery in varying applications. As described with respect to FIGS. 11A-11D, 11G, and 11H, proximal hole 1548, distal hole 1546B, and distal hole 1546B can be spaced along the length of adjustable contrast tube 1542 to optimize delivery of the contrast agent to targeted regions of interest. In some examples, proximal hole 1548 can be spaced from distal hole 1546B by approximately 1 in. (25.4 mm) and distal hole 1546B can be spaced from distal hole 1546A by approximately 0.1 in. (2.54 mm). It will be understood by one of ordinary skill in the art that the spacing between proximal hole 1548, distal hole 1546A, and distal hole 1546B can be modified as needed to provide targeted contrast delivery in varying applications. Distal terminal end 1550 is closed. As such, all contrast agent is delivered from adjustable contrast tube 1542 via proximal hole 1548 and distal holes 1546A and 1546B.

As illustrated in FIGS. 12A-12D, adjustable contrast tube 1542 is slidably received in lumen 1522 and traversable between a plurality of positions to deliver the contrast agent to targeted regions, for example, toward distal arm 1508A and proximal arm 1510A. Adjustable contrast tube 1542 can be positioned in bridge 1504 in assembly of delivery device 1540 and advanced and/or retracted to provide targeted delivery of the contrast agent during deployment of shunt device 1506. FIG. 12B illustrates adjustable contrast tube 1542 in a first position; FIG. 12C illustrates adjustable contrast tube 1542 in a second position; FIG. 12D illustrates adjustable contrast tube 1542 in a third position. The position of adjustable contrast tube 1542 is not limited to the positions illustrated. The use of the terms "first," "second," and "third" does not limit the order in which adjustable contrast tube 1542 is positioned for delivery of the contrast agent.

As described with respect to delivery device 1500 of FIGS. 11A-11D, a user can position adjustable contrast tube 1542 in each of the first, second, and third positions by advancing and/or retracting adjustable contrast tube 1542 from the proximal end of adjustable contrast tube 1542. A positioning mechanism (not shown) can be included, for example, on a proximal handle of the delivery catheter (e.g., handle 216 of FIG. 6) to allow a user to manipulate adjustable contrast tube 1542 and locate adjustable contrast tube 1542 at each of the first, second, and third positions. Adjustable contrast tube 1542 can be positioned at each of the first, second, and third positions to deliver the contrast agent to targeted regions. Adjustable contrast tube 1542 can be positioned in one of the first, second, or third positions upon assembly such that adjustable contrast tube 1542 is in position for delivery of the contrast agent upon delivery of shunt device 1506. The user can slide adjustable contrast tube 1542 to the next of the desired first, second, and third positions to deliver the contrast agent to another targeted region. The user can again slide adjustable contrast tube 1542 to the last of the desired first, second, and third positions to deliver the contrast agent to yet another targeted region. Contrast can be delivered from adjustable contrast tube 1542 from each of the first, second, and third positions any number of times to confirm proper placement or tissue capture of shunt device 1506.

Proximal hole 1548 and distal holes 1546A and 1546B are aligned along the length of adjustable contrast tube 1542 such that each of proximal hole 1548 and distal holes 1546A and 1546B can be aligned with exit hole 1526 by sliding adjustable contrast tube 1542 in lumen 1522 forward and backward without twisting adjustable contrast tube 1542. Each of proximal hole 1548 and distal holes 1546A and 1546B can have a diameter substantially matching a diameter of exit hole 1526. The diameter of proximal hole 1548, distal holes 1546A and 1546B, and exit hole 1526 can be, for example, approximately 0.03 in. (0.762 mm) to promote the flow of the contrast agent during injection without requiring application of excessive force by the user.

As illustrated in FIG. 12B, distal hole 1546A is aligned with exit hole 1526 of bridge 1504 when adjustable contrast tube 1542 is in the first position. Distal hole 1546A is open to exit hole 1526 and thereby the outer surface of bridge 1504 to allow flow of the contrast agent when adjustable contrast tube 1542 is in the first position. Proximal hole 1548 and distal hole 1546B are located in lumen 1522 when adjustable contrast tube 1542 is in the first position and are effectively closed such that the contrast agent is preferentially delivered through distal hole 1546A and exit hole 1526. Although there is a small gap between the outer diameter of adjustable contrast tube 1542 and the inner diameter of lumen 1522, the gap is generally too small for the contrast agent to leak from proximal hole 1548 and distal hole 1546B when contrast agent is injected into adjustable contrast tube 1542. The contrast agent is thereby preferentially delivered through distal hole 1546A and exit hole 1526 when adjustable contrast tube 1542 is in the first position. As illustrated in FIG. 12B, upon delivery of shunt device 1506, exit hole 1526 is located in the coronary sinus CS and positioned to deliver contrast agent toward proximal arm 1510A of shunt device 1506 when adjustable contrast tube 1542 is in the first position. **In** the first position, no openings from adjustable contrast tube 1542 are positioned in the left atrium LA, and all injected contrast agent is preferentially delivered to coronary sinus CS when adjustable contrast tube 1542 is in the first position.

As illustrated in FIG. 12C, distal holes 1546A and 1546B are positioned in side opening 1528 and open to allow flow of the contrast agent when adjustable contrast tube 1542 is in the second position. Proximal hole 1548 is aligned with exit hole 1526 and open to allow flow of the contrast agent when adjustable contrast tube 1542 is in the second position. As illustrated in FIG. 12C, exit hole 1526 is positioned in the coronary sinus CS and side opening 1528 is positioned in the left atrium LA following deployment of shunt device 1506. Proximal hole 1548 is positioned to direct the contrast agent into the coronary sinus CS and toward proximal arm 1510A of shunt device 1506 when adjustable contrast tube 1542 is in the second position. Distal holes 1546A and 1546B are positioned to direct the contrast agent into the left atrium LA and toward distal arm 1508A of shunt device 1506 when adjustable contrast tube 1542 is in the second position. **In** the second position, the injected contrast agent is delivered to both coronary sinus CS and left atrium LA when adjustable contrast tube 1542 is in the second position.

As illustrated in FIG. 12D, distal holes 1546A and 1546B are positioned in or adjacent to side opening 1528 and open to allow flow of the contrast agent when adjustable contrast tube 1542 is in the third position. Proximal hole 1548 is positioned in lumen 1522 proximal to exit hole 1526 and effectively closed to flow of the contrast agent when adjustable contrast tube 1542 is in the third position. As illustrated in FIG. 12D, adjustable contrast tube 1542 is advanced between the second and third positions such that proximal hole 1548 is positioned distal to exit hole 1526. Alternatively, or in addition to the position shown in FIG. 12D, adjustable contrast tube 1542 can be retracted such that proximal hole 1548 is positioned in lumen 1522 proximal to exit hole 1526. As illustrated in FIG. 12A, side opening 1528 is positioned in left atrium LA following deployment of shunt device 1506. Distal holes 1546A and 1546B are positioned to direct the contrast agent into the left atrium LA and toward distal arm 1508A of shunt device 1506 when adjustable contrast tube 1542 is in the third position. **In** the third position, no openings in adjustable contrast tube 1542 are positioned in coronary sinus CS and all injected contrast agent is preferentially delivered to the left atrium LA when adjustable contrast tube 1542 is in the third position.

Adjustable contrast tube 1542 can be formed, for example, of a shape-memory material configured to assume a curved shape when distal tip 1544 is unconfined and free to flex.

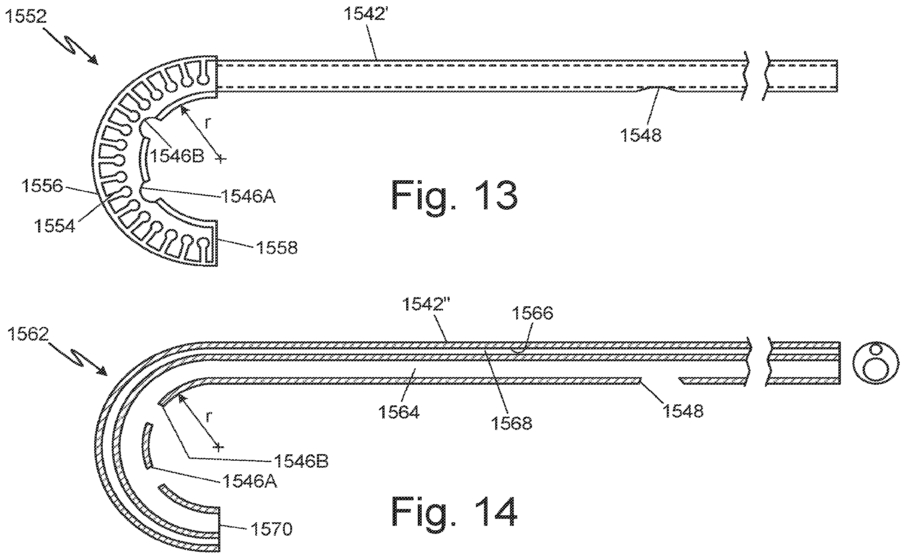

FIG. 13 is a side view of one example of adjustable contrast tube 1542 of FIGS. 12A-12D with distal tip 1544 in a curved orientation. FIG. 13 shows adjustable contrast tube 1542' having distal tip 1552, distal holes 1546A and 1546B, proximal hole 1548, slits 1554, sleeve 1556, and terminal end 1558. Adjustable contrast tube 1542' is formed of a laser cut nitinol hypotube with a sheathed distal tip 1552. Slits 1554 can be laser cut and patterned to allow flexing of distal tip 1552. Slits 1554 extend a partial circumference of distal tip 1552 and are spaced along a length of distal tip 1552 to promote curvature of distal tip 1552. The amount and spacing of slits 1554 can be optimized to provide a desired curvature of distal tip 1552. Distal tip 1552 can have a radius of curvature r, for example, of approximately 0.15 in. (3.81 mm) to direct the contrast agent toward the region of interest. Slits 1554 can be formed on opposite side walls of distal tip 1552 (not shown) as needed for flexibility. Adjustable contrast tube 1542' can be heat treated to shape set the curved orientation of distal tip 1552 when distal tip 1552 is outside of lumen 1522. Distal tip 1552 formed of laser cut nitinol hypotube can include sleeve 1556. Sleeve 1556 can be configured to seal the laser cut nitinol hypotube and/or to close terminal end 1558 of adjustable contrast tube 1542'. Sleeve 1556 can include holes sized to match distal holes 1546A and 1546B and positioned to align with distal holes 1546A and 1546B. Sleeve 1556 is flexible and configured to move with distal tip 1552. Sleeve 1556 can be, for example, a Pebax^{®} thermoplastic elastomer or other suitable flexible and biocompatible material known in the art. **In** some examples, adjustable contrast tube 1502' can have a minimum inner diameter of 0.042 in. (1.07 mm) to deliver the contrast agent with reduced resistance.

FIG. 14 is a cross-sectional view of another example of adjustable contrast tube 1542 of FIGS. 12A-12D with distal tip 1562 in a curved orientation. FIG. 14 shows adjustable contrast tube 1542" having distal tip 1562, distal holes 1546A and 1546B, proximal hole 1548, primary lumen 1564, secondary lumen 1566, shape set wire 1568, and terminal end 1570. Adjustable contrast tube 1542" is formed of a polyamide braid with a Pebax^{®} thermoplastic elastomer shape set distal tip 1562. Adjustable contrast tube 1542" can be formed through an extrusion process. Adjustable contrast tube 1542" includes primary lumen 1564 for delivery of the contrast agent and secondary lumen 1566 though which shape set wire 1568 is disposed. Shape set wire 1568 is contained in the secondary lumen. In some examples, primary lumen 1564 can have a minimum inner diameter of 0.042 in. (1.07 mm) to deliver the contrast agent with reduced resistance. Shape set wire 1568 can provide a spine that assumes a curved shape when free of restriction (i.e., outside of lumen 1522). Shape set wire 1568 can be a nitinol wire. Other combinations of materials including shape-memory materials known in the art suitable for delivering a contrast agent are contemplated. Adjustable contrast tube 1542" can be heat treated to shape set the curved orientation of distal tip 1562 when distal tip 1562 is outside of lumen 1522. Distal tip 1562 can have a radius of curvature r, for example, of approximately 0.15 in. (3.81 mm) to direct the contrast agent toward the region of interest. Adjustable contrast tube 1542" can be manufactured with a closed distal terminal end 1570.

Adjustable contrast tubes 1542' and 1542" maintain a straight orientation when confined in lumen 1522. Distal tips 1552, 1562 begin to assume a curved orientation as illustrated in FIGS. 12C and 12D as adjustable contrast tubes 1542' and 1542" exit lumen 1522 to side opening 1528 or as an outer sheath of bridge 1504 (e.g., outer sheath 218 of delivery catheter 200) is removed to deploy shunt device 1506. As illustrated in FIGS. 12A, 12C, and 12D, distal tip 1544 curves outward from bridge 1504 when positioned in side opening 1528 such that distal holes 1546A and 1546B face toward proximal hole 1548 and, importantly, toward tissue wall TW in left atrium LA to confirm tissue capture of shunt device 1506 upon delivery, as illustrated in FIGS. 12C and 12D.

The contrast agent can be delivered via adjustable contrast tube 1502 or 1542 following delivery of shunt device 1506. As previously described, both adjustable contrast tube 1502 and 1542 are configured to deliver the contrast agent to regions of interest. As illustrated in FIGS. 11B-11D and 12B-12D, adjustable contrast tubes 1502 and 1542 can be configured to confirm tissue capture of shunt device 1506 when deployed, for example, between tissue wall TW of the coronary sinus CS and left atrium LA. In some instances, shunt device 1506 can be improperly seated with both distal arm 1508A and proximal arm 1510A disposed in the left atrium LA or in the coronary sinus CS. Improper positioning of shunt device 1506 can be observed by imaging the region of shunt device 1506 via fluoroscopy during delivery of the contrast agent, which can illuminate distal arm 1508A and proximal arm 1510A. If shunt device 1506 is properly seated, tissue wall TW (evidenced by lack of the contrast agent) can be observed between distal arm 1508A and proximal arm 1510A, which are illuminated by the contrast agent. If shunt device 1506 is improperly seated such that tissue wall TW are not captured between distal arm 1508A and proximal arm 1510A, tissue wall TW will not be visible between distal arm 1508A and proximal arm 1510A as evidenced by the presence of the contrast agent between distal arm 1508A and proximal arm 1510A.

Distal arm 1508A and proximal arm 1510A of shunt device 1506 are most susceptible to mis-seating. As such, adjustable contrast tubes 1502 and 1542 are oriented to direct the contrast agent toward distal arm 1508A and proximal arm 1510A. As previously discussed, adjustable contrast tubes 1502 and 1542 can be moved between first, second, and third positions to deliver the contrast agent to the regions of interest, e.g., left atrium LA only, coronary sinus CS only, and left atrium LA and coronary sinus CS simultaneously. Adjustable contrast tubes 1502 and 1542 used in conjunction with bridge 1504 can target delivery of the contrast agent to regions of interest to improve confirmation of tissue capture of shunt device 1506 following deployment. Adjustable contrast tubes 1502 and 1542 do not require blood flow to move the contrast agent to the regions of interest and the flow of the contrast agent is less susceptible to issues relating to dilution. The contrast agent can be directed though exit hole 1526 directly toward proximal arm 1510A and from distal holes 1514A and 1514B or 1546A and 1546B when positioned in side opening 1528 directly toward distal arm 1508A to effectively illuminate proximal arm 1510A and distal arm 1508A.

### DELIVERY DEVICE 1600

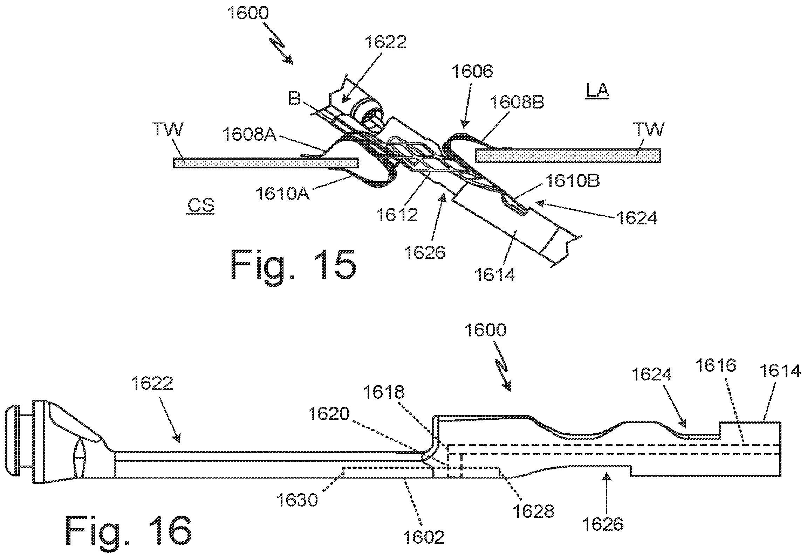

FIG. 15 is a perspective view of delivery device 1600 for delivering and placing shunt device 1606 within a human body and confirming tissue capture of shunt device 1606 with contrast delivery via channel 1602. FIG. 16 is a side view of delivery device 1600 of FIG. 15 with contrast delivery channel 1602. FIGS. 15 and 16 will be discussed together. Delivery device 1600, contrast deliver channel 1602 (shown in FIG. 16), shunt device 1606 (shown in FIG. 15), distal arms 1608A and 1608B (shown in FIG. 15), proximal arms 1610A and 1610B (shown in FIG. 15), central flow tube 1612 (shown in FIG. 15), tissue wall TW (shown in FIG. 15), coronary sinus CS (shown in FIG. 15), left atrium LA (shown in FIG. 15), bridge 1614, lumen 1616 (shown in FIG. 16), distal end 1618 (shown in FIG. 16), feed port 1620 (shown in FIG. 16), pocket 1622, notch 1624, and side opening 1626 are shown.

Delivery device 1600 is one example of delivery catheter 200 shown in FIG. 7B to deliver shunt device 1606 and confirm placement of shunt device 1606 in tissue wall TW. Shunt device 1606 can be deployed as described in method 300 shown in FIGS. 8A, 8B, and 9A-9Q and described with respect thereto with a modification of the step of contrast delivery for tissue capture confirmation shown in FIG. 9O.

Shunt device 1606 can substantially the same as or similar to shunt devices 100 and 100' illustrated in FIGS. 3A, 3B, 4, and 5 and described with respect thereto. Shunt device 1606 includes distal arms 1608A and 1608B, proximal arms 1610A and 1610B, and central flow tube 1612.

Delivery device 1600 includes bridge 1614. Bridge 1614 can be substantially similar to bridge 222 of delivery catheter 200 shown in FIG. 7B with the addition of channel 1602 and relocation of pocket 1622. Bridge 1614 includes lumen 1616 with distal end 1618, feed port 1620, channel 1602, pocket 1622, notch 1624, and side opening 1626. Distal end 1618 of lumen 1616 is closed. Feed port 1620 connects lumen 1616 to channel 1602. Channel 1602 is open to an outer surface of bridge 1614.

Pocket 1622 is positioned on a side of bridge 1614. Pocket 1622 cuts into the body of bridge 1614 and extends lengthwise along the side of bridge 1614. Pocket 1622 can be configured to seat a sensor of shunt device 1606 (i.e., sensor 150', illustrated in FIG. 3) for delivery into a human body. Pocket 1622 is positioned adjacent to distal end 1618 of lumen 1616. **In** the orientation shown, pocket 1622 is positioned on a top side of bridge 1614.

Notch 1624 is positioned proximal to pocket 1622 on a top side of bridge 1614. Notch 1624 can be substantially the same as notch 229 of bridge 222 as described with respect to FIG. 7B. Notch 1624 cuts into the body of bridge 1614 opposite side opening 1626. Notch 1624 can be configured to seat proximal arm 1610B of shunt device 1606.

Side opening 1626 can be substantially similar to side opening 228 as described with respect to FIG. 7B. Side opening 1626 cuts into the body of bridge 1614 and extends lengthwise along the side of bridge 1614. Side opening 1626 terminates at a location proximal to channel 1602. **In** the orientation shown, side opening 1626 is positioned on a bottom side of bridge 1614 opposite notch 1624. Side opening 1626 can receive actuation rod 226, as described with respect to delivery catheter 200 as shown in FIGS. 6-7B. Actuation rod 226 can extend into side opening 1626 in bridge 1614 and connect to proximal arm 1610A of shunt device 1606.

Channel 1602 is configured to deliver a contrast agent to a region of interest to confirm proper placement or tissue capture of shunt device 1606. As described further herein, channel 1602 is configured to bridge locations in which distal arm 1608A and proximal arm 1610A are positioned (e.g., left atrium LA and coronary sinus CS) and deliver the contrast agent therebetween to confirm the presence of tissue wall TW. The location of channel 1602 is indicated by box B in FIG. 15.

Lumen 1616 (shown in phantom in FIG. 16) is configured to deliver a contrast agent. Lumen 1616 extends lengthwise through at least a portion of bridge 1614. Lumen 1616 can be one of a plurality of lumens disposed in bridge 1614 as previously described with respect to bridge 222 shown in FIGS. 6-7B. Lumen 1616 has distal end 1618, which is closed. Distal end 1618 is disposed proximal to pocket 1622 and spaced from pocket 1622. Lumen 1616 can be substantially aligned with an innermost extent of pocket 1622. For example, as illustrated in FIG. 16, pocket 1622 can be cut into the body of bridge 1614 such that an innermost extent of pocket 1622 is substantially aligned with an innermost extent of lumen 1616, identified as the location from which feed port 1620 extends. Lumen 1616 can be molded with bridge 1614 and configured to carry the contrast agent. Lumen 1616 has an inner diameter sized to accommodate delivery of the contrast agent without application of excessive force during injection by a user. **In** some examples, lumen 1616 can have a minimum inner diameter of approximately 0.042 in. (1.07 mm).

Feed port 1620 (shown in phantom in FIG. 16) fluidly connects lumen 1616 to channel 1602. Feed port 1620 can be disposed adjacent to distal end 1618. Feed port 1620 can extend substantially perpendicular to lumen 1616 such that the intersection of lumen 1616 at distal end 1618 and feed port 1620 has an L shape. Feed port 1620 can be substantially cylindrical. Feed port 1620 can have an inner diameter substantially matching an inner diameter of lumen 1616 to accommodate flow of the contrast agent with injection. Feed port 1620 can be co-molded with lumen 1616.

Channel 1602 (shown in phantom in FIG. 16) includes oppositely disposed proximal channel end 1628 and distal channel end 1630. Channel 1602 extends lengthwise along an outer surface of bridge 1614 between proximal channel end 1628 and distal channel end 1630. Channel 1602 forms a trough in a side wall of bridge 1614 open to feed port 1620 and the outer surface of bridge 1614. Feed port 1620 can be positioned adjacent tissue wall TW during delivery of the contrast agent, and tissue wall TW can interrupt flow of the contrast agent from bridge 1614 in the area of feed port 1620. Channel 1602 can have a width and shape configured to channel the contrast agent between proximal channel end 1628 and distal channel end 1630, such that flow of the contrast agent is distributed along channel 1602 and is not limited to the location of feed port 1620. Channel 1602 can be disposed on a side of bridge 1614 opposite pocket 1622. Feed port 1620 fluidly connects lumen 1616 to channel 1602. Feed port 1620 can be positioned between distal channel end 1630 and proximal channel end 1628. As illustrated in FIG. 16, feed port 1620 can be disposed closer to proximal channel end 1628 than distal channel end 1630 to accommodate the spacing between lumen 1616 and pocket 1622. As illustrated in FIG. 15, bridge 1614 is positioned through central flow tube 1612 of shunt device 1606. Channel 1602 is disposed to substantially align with central flow tube 1612. Channel 1602 has a length sufficient to deliver the contrast agent to both coronary sinus CS and left atrium LA. **In** some examples, channel 1602 can have a length greater than a length of central flow tube 1612. Channel 1602 can have a length equal to or greater than a thickness of tissue wall TW (e.g., 2 mm). Channel 1602 can be disposed to extend beyond a location from which proximal arm 1610A and distal arm 1608A extend outward from bridge 1614 as illustrated in FIG. 15. When shunt device 1606 is properly deployed (e.g., between left atrium LA and coronary sinus CS), channel 1602 spans both sides of or across tissue wall TW, such that distal channel end 1630 is positioned in the left atrium LA and proximal channel end 1628 is positioned in the coronary sinus CS.

During or following deployment of shunt device 1606, the contrast agent is injected through lumen 1616, feed port 1620, and channel 1602 of bridge 1614 and through central flow tube 1612 of shunt device 1606 towards proximal arm 1610A and distal arm 1608A. The contrast agent flows outward from bridge 1614 and perpendicular to bridge 1614 along the length of channel 1602. The contrast agent flows through openings in a wall of central flow tube 1612 (e.g., openings 106 in end portion 122A, illustrated in FIG. 3A). A region including shunt device 1606 can be imaged via fluoroscopy during injection of the contrast agent. When shunt device 1606 is properly positioned, the plume of contrast agent delivered from bridge 1614 and spanning the length of central flow tube 1612 and proximal arm 1610A and distal arm 1608A is interrupted by tissue wall TW. For example, dark regions indicating the presence of contrast are interrupted by a light region indicating the presence of tissue wall TW. **In** other words, the contrast agent is visible on opposite sides of tissue wall TW when shunt device 1606 is properly positioned. If shunt device 1606 is improperly positioned, a plume of uninterrupted contrast agent will be visible between proximal arm 1610A and distal arm 1608A. If shunt device 1606 is improperly positioned, the flow of the contrast agent from feed port 1620 is less likely to be interrupted by tissue wall TW such that the contrast agent is preferentially delivered from feed port 1620, which may be visible in imaging the injection of the contrast agent.

Contrast delivery channel 1602 can be used to deliver a contrast agent from an extended location along a length of bridge 1614 to direct the contrast agent to multiple target locations, including, for example, both left atrium LA and coronary sinus CS, to improve confirmation of tissue capture of shunt device 1606 following deployment. Contrast delivery channel 1602 does not require blood flow to move the contrast agent to the regions of interest and the flow of the contrast agent is less susceptible to issues relating to dilution. The contrast agent can be directed though channel 1602 toward proximal arm 1610A and distal arm 1608A to effectively illuminate proximal arm 1610A and distal arm 1608A and the region therebetween.

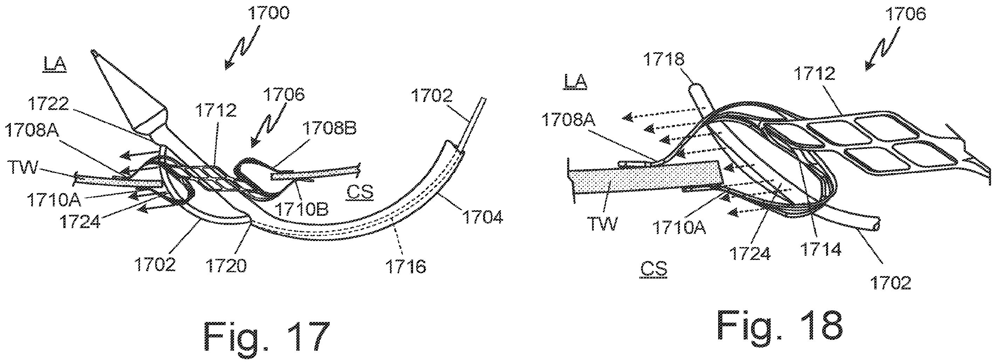

### DELIVERY DEVICE 1700 (FIGS. 17-18)

FIG. 17 is a perspective view of delivery device 1700 for delivering and placing a shunt device within a human body and confirming tissue capture of the shunt device with contrast delivery via contrast tube 1702. FIG. 18 is a simplified view of contrast tube 1702 of FIG. 17 illustrating delivery of a contrast agent (shown by flow arrows). FIGS. 17 and 18 will be discussed together. Delivery device 1700 (shown in FIG. 17), contrast tube 1702, bridge 1704 (shown in FIG. 17), shunt device 1706, distal arms 1708A and 1708B, proximal arms 1710A and 1710B, central flow tube 1712, exterior surface 1714 (shown in FIG. 18), tissue wall TW, coronary sinus CS, left atrium LA, lumen 1716 (shown in FIG. 17), distal end 1718 (shown in FIG. 18), side opening 1720 (shown in FIG. 17), distal attachment 1722 (shown in FIG. 17), and slit opening 1724 are shown.

Delivery device 1700 is one example of delivery catheter 200 shown in FIG. 7B to deliver shunt device 1706 and confirm placement of shunt device 1706 in tissue wall TW. Shunt device 1706 can be deployed as described in method 300 shown in FIGS. 8A, 8B, and 9A-9Q and described with respect thereto with a modification of the step of contrast delivery for tissue capture confirmation shown in FIG. 9O.

Shunt device 1706 can be substantially similar to shunt devices 100 and 100' illustrated in FIGS. 3A, 3B, 4, 5 and described with respect thereto, with modification of struts 104 in proximal arm 132 and distal arm 130 and/or modification of distal arm 130 to allow positioning of contrast tube 1702 along exterior surface 1714 of central flow tube 1712. Modifications of shunt device 1706 are described further herein.

Delivery device 1700 includes bridge 1704. Bridge 1704 can be substantially similar to bridge 222 of delivery catheter 200 shown in FIG. 7B with the addition of lumen 1716 configured to receive contrast tube 1702. Lumen 1716 extends lengthwise through bridge 1704 and opens to side opening 1720. Side opening 1720 can be substantially the same as side opening 228 as described with respect to FIG. 7B. Side opening 1720 cuts into the body of bridge 1704 and extends lengthwise along the side of bridge 1704. Side opening 1720 is positioned adjacent to distal end of lumen 1716. In some examples, side opening 1720 can be configured to seat a sensor of shunt device 1706 (i.e., sensor 150', illustrated in FIG. 3) for delivery into a human body.

Delivery device 1700 can be substantially similar to delivery devices 1500 and 1540 illustrated in FIGS. 11A and 12A and described with respect thereto, with a modified contrast tube 1702 having slit opening 1724. Contrast tube 1702 with slit opening 1724 is configured to deliver a contrast agent to a region of interest to confirm proper placement or tissue capture of shunt device 1706. As described further herein, slit opening 1724 is configured similarly to channel 1602 of delivery device 1600 to bridge locations in which distal arm 1708A and proximal arm 1710A are positioned (e.g., left atrium LA and coronary sinus CS) and deliver the contrast agent therebetween to confirm the presence of the tissue wall TW. **In** contrast to channel 1602, slit opening 1724 is positioned outside of central flow tube 1712 such that flow of the contrast agent is uninterrupted by struts of central flow tube 1712.

Lumen 1716 (shown in phantom) is configured to receive contrast tube 1702. Lumen 1716 extends lengthwise through at least a portion of bridge 1704. Lumen 1716 can be one of a plurality of lumens disposed in bridge 1704 as previously described with respect to bridge 222 of delivery catheter 200. Lumen 1716 has a distal end opening to side opening 1720. Lumen 1716 has an inner diameter sized to accommodate a sliding motion of contrast tube 1702 to allow a user to advance and retract contrast tube 1702 through lumen 1716 during contrast delivery procedures. **In** some examples, lumen 1716 can have an inner diameter approximately 0.01 in. (0.254 mm) larger than an outer diameter of contrast tube 1702. **In** some examples, lumen 1716 can have an inner diameter of approximately N 0.055 in. (1.4 mm).

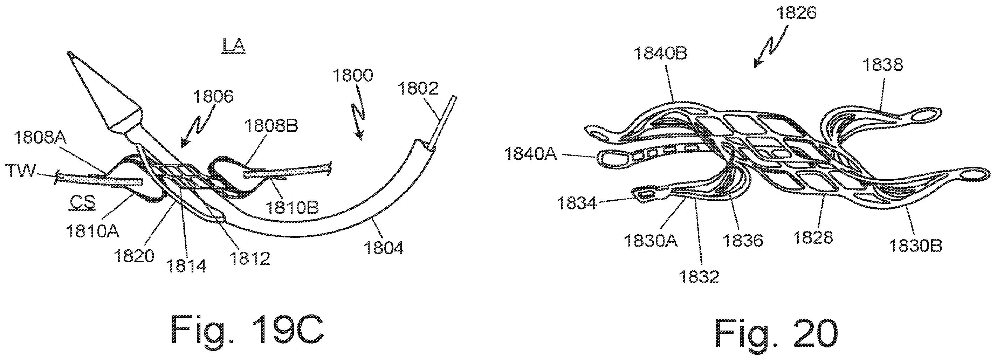

Contrast tube 1702 is disposed in lumen 1716 and extends outward from lumen 1716 at side opening 1720. Contrast tube 1702 is slidably received in lumen 1716. Contrast tube 1702 can be formed of a flexible material (e.g., polyamide braided tube) configured for delivering a contrast agent. Contrast tube 1702 is disposed external to central flow tube 1712 of shunt device 1706. Contrast tube 1702 is disposed adjacent to and extends along exterior surface 1714 of central flow tube 1712. Contrast tube 1702 extends along a length of central flow tube 1712 (e.g., between proximal arm 1710A and distal arm 1708A). Contrast tube 1702 can be disposed through an opening of proximal arm 1710A, such as opening 106 between struts 104 in shunt device 100 in FIG. 3A, to allow positioning of contrast tube 1702 along central flow tube 1712. Contrast tube 1702 can be disposed through an opening of distal arm 1710A to allow positioning of contrast tube 1702 along central flow tube 1712. As discussed further with respect to FIG. 20, shunt device 1706 can be configured to optimize placement of contrast tube 1702 relative to central flow tube 1712, proximal arm 1710A, and distal arm 1708A. For example, struts in proximal arm 1710A and/or distal arm 1708A can be arranged to provide a centrally located opening through proximal arm 1710A and/or distal arm 1708A. **In** some examples, distal arm 1708A can be split into two arms between which contrast tube 1702 can be located.

Contrast tube 1702 includes slit opening 1724 configured to deliver the contrast agent. Contrast tube 1702 is closed at distal end 1718 such that all contrast delivery is through slit opening 1724. A portion of contrast tube 1702 including slit opening 1724 is disposed adjacent to exterior surface 1714 of central flow tube 1712. Slit opening 1724 extends along a length of contrast tube 1702. Slit opening 1724 faces away from exterior surface 1714 of central flow tube 1712 toward terminal ends of proximal arm 1710A and distal arm 1708A. Slit opening 1724 can be a cut into a side of contrast tube 1702 providing an opening sized to deliver the contrast agent from an extended location along the length of contrast tube 1702. For example, slit opening 1724 can extend substantially a full length of central flow tube 1712 to deliver the contrast agent toward each of proximal arm 1710A and distal arm 1708A following delivery of shunt device 1706. When shunt device 1706 is properly deployed (e.g., between the left atrium LA and coronary sinus CS), slit opening 1724 spans both sides of or across tissue wall TW, such that a portion of slit opening 1724 is positioned in the left atrium LA and a portion of slit opening 1724 is positioned in the coronary sinus CS.

**In** some examples, bridge 1704 can include distal attachment 1722. Distal attachment 1722 is positioned along the exterior wall adjacent to a distal end of contrast tube 1702. Distal attachment 1722 is configured to retain the distal end of contrast tube 1702 during contrast delivery to ensure proper placement of contrast tube 1702 along central flow tube 1712. Distal attachment 1722 can be, for example, a pocket or sheath or other mechanism configured to hold the distal end of contrast tube 1702. Distal attachment 1722 can provide temporary retention of contrast tube 1702 thereby allowing removal of contrast tube 1702 following injection of the contrast agent. Contrast tube 1702 can be positioned through openings of proximal arm 1710A and distal arm 1708A and along central flow tube 1712 of shunt device 1706 in assembly. Because contrast tube 1702 is properly positioned in assembly, distal attachment 1722 may not be necessary in all applications.

Following deployment of shunt device 1706, the contrast agent is injected through contrast tube 1702 and slit opening 1724 towards proximal arm 1710A and distal arm 1708A of shunt device 1706, as illustrated by flow arrows in FIGS. 17 and 18. The contrast agent flows outward from contrast tube 1702 along the length of slit opening 1724. Because contrast tube 1702 is located external to central flow tube 1712, the flow of the contrast agent is uninterrupted by struts of central flow tube 1712. A region including shunt device 1706 can be imaged via fluoroscopy during or following injection of the contrast agent. When shunt device 1706 is properly positioned, the plume of contrast agent delivered from contrast tube 1702 and spanning the length of central flow tube 1712 and proximal arm 1710A and distal arm 1708A is interrupted by tissue wall TW. For example, dark regions indicating the presence of contrast are interrupted by a light region indicating the presence of tissue wall TW. In other words, the contrast agent is visible on opposite sides of tissue wall TW when shunt device 1706 is properly positioned. If shunt device 1706 is improperly positioned, a plume of uninterrupted contrast agent will be visible between proximal arm 1710A and distal arm 1708A.

Contrast tube 1702 can be used to deliver a contrast agent from an extended location along a length of contrast tube 1702 to direct the contrast agent to multiple target locations, including, for example, both left atrium LA and coronary sinus CS, to improve confirmation of tissue capture of shunt device 1706 following deployment. Contrast tube 1702 does not require blood flow to move the contrast agent to the regions of interest and the flow of the contrast agent is less susceptible to issues relating to dilution. The contrast agent can be directed though slit opening 1724 directly toward proximal arm 1710A and distal arm 1708A uninterrupted by central flow tube 1712 to effectively illuminate proximal arm 1710A and distal arm 1708A and the region therebetween.

### DELIVERY DEVICE 1800

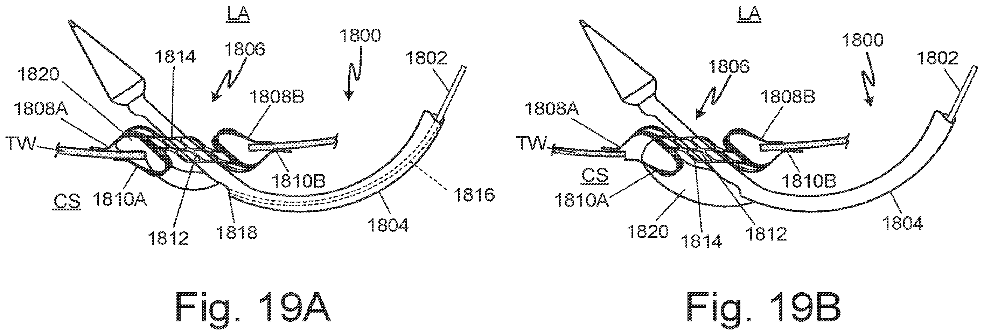

FIG. 19A is a perspective view of delivery device 1800 for delivering and placing a shunt device within a human body and confirming tissue capture of the shunt device with contrast delivery via an inflatable contrast balloon. FIG. 19B is a perspective view of delivery device 1800 of FIG. 19A illustrating improper tissue capture. FIG. 19C is a perspective view of delivery device 1800 of FIG. 19A with the contrast delivery balloon deflated. FIGS. 19A-19C will be discussed together. Delivery device 1800, contrast tube 1802, bridge 1804, shunt device 1806, distal arms 1808A and 1808B, proximal arms 1810A and 1810B, central flow tube 1812, exterior surface 1814, tissue wall TW, coronary sinus CS, left atrium LA, lumen 1816 (shown in FIG. 19A), side opening 1818 (shown in FIG. 19A), and inflatable contrast balloon 1820, are shown.

Delivery device 1800 is one example of delivery catheter 200 shown in FIG. 7B to deliver shunt device 1806 and confirm placement of shunt device 1806 in tissue wall TW. Shunt device 1806 can be deployed as described in method 300 shown in FIGS. 8A, 8B, and 9A-9Q and described with respect thereto with a modification of the step of contrast delivery for tissue capture confirmation shown in FIG. 9O.

Shunt device 1806 can be substantially similar to shunt devices 100 and 100' illustrated in FIGS. 3A, 3B, 4, 5 and described with respect thereto, with modification of struts 104 in proximal arm 132 and distal arm 130 and/or modification of distal arm 130 to allow positioning of contrast tube 1802 along exterior surface 1814 of central flow tube 1812. Modifications of shunt device 1806 are described further herein.

Delivery device 1800 includes bridge 1804. Bridge 1804 can be substantially similar to bridge 222 of delivery catheter 200 shown in FIG. 7B with the addition of lumen 1816 configured to receive contrast tube 1802. Lumen 1816 extends lengthwise through bridge 1804 and opens to side opening 1818. Side opening 1818 can be substantially the same as side opening 228 as described with respect to FIG. 7B. Side opening 1818 cuts into the body of bridge 1804 and extends lengthwise along the side of bridge 1804. Side opening 1818 is positioned adjacent to distal end of lumen 1816. In some examples, side opening 1818 can be configured to seat a sensor of shunt device 1806 (i.e., sensor 150', illustrated in FIG. 3) for delivery into a human body.

Delivery device 1800 can be substantially similar to delivery device 1700 illustrated in FIGS. 17 and 18 and described with respect thereto, with the replacement of contrast tube 1702 and slit opening 1724 with contrast tube 1802 and inflatable contrast balloon 1820. Contrast tube 1802 with inflatable contrast balloon 1820 is configured to deliver a contrast agent to a region of interest to confirm proper placement or tissue capture of shunt device 1806 without releasing the contrast agent into the human body. As described further herein, inflatable contrast balloon 1820 is configured to expand with the contrast agent to bridge locations in which distal arm 1808A and proximal arm 1810A are positioned (e.g., left atrium LA and coronary sinus CS) to confirm the presence of the tissue wall TW without infusing the contrast agent into the patient.

Lumen 1816 (shown in phantom) is configured to receive contrast tube 1802 with contrast balloon 1820. Lumen 1816 extends lengthwise through at least a portion of bridge 1804. Lumen 1816 can be one of a plurality of lumens disposed in bridge 1804 as previously described with respect to bridge 222 of delivery catheter 200. Lumen 1816 has an opening at a distal end to side opening 1818. Lumen 1816 has an inner diameter sized to accommodate a sliding motion of contrast tube 1802 with inflatable contrast balloon 1820 within lumen 1816 to allow a user to advance and retract contrast tube 1802 through lumen 1816 during contrast delivery procedures. In some examples, lumen 1816 can have an inner diameter approximately 0.01 in. (0.254 mm) larger than an outer diameter of contrast tube 1802. **In** some examples, lumen 1816 can have an inner diameter of approximately 0.055 in. (1.4 mm).

Contrast tube 1802 is disposed in lumen 1816 and extends outward from lumen 1816 at side opening 1818. Contrast tube 1802 is slidably received in lumen 1816. Contrast tube 1802 can be formed of a flexible material (e.g., polyamide braided tube) configured for delivering a contrast agent. Contrast tube 1802 is disposed external to central flow tube 1812 of shunt device 1806. Contrast tube 1802 is disposed adjacent to and extends along exterior surface 1814 of central flow tube 1812. Contrast tube 1802 extends along a length of central flow tube 1812 (e.g., between proximal arm 1810A and distal arm 1808A). Contrast tube 1802 can be disposed through an opening of proximal arm 1810A, such as opening 106 between struts 104 in shunt device 100 in FIG. 3A, to allow positioning of contrast tube 1802 along central flow tube 1812. Contrast tube 1802 can be disposed through an opening of distal arm 1808A to allow positioning of contrast tube 1802 along central flow tube 1812. As discussed further with respect to FIG. 20, shunt device 1806 can be configured to optimize placement of contrast tube 1802 relative to central flow tube 1812, proximal arm 1810A, and distal arm 1808A. For example, struts in proximal arm 1810A and/or distal arm 1808A can be arranged to provide a centrally located opening through proximal arm 1810A and/or distal arm 1808A. **In** some examples, distal arm 1808A can be split into two arms between which contrast tube 1802 can be located.

Contrast tube 1802 includes inflatable contrast balloon 1820. Inflatable contrast balloon 1820 is disposed at a distal end of contrast tube 1802 and open to contrast tube 1802 to receive the contrast agent. Inflatable contrast balloon 1820 is closed at a distal end to contain the contrast agent. Inflatable contrast balloon 1820 is disposed adjacent to exterior surface 1814 of central flow tube 1812. Inflatable contrast balloon 1820 extends along a length of contrast tube 1802. Inflatable contrast balloon 1820 can extend a full length of central flow tube 1812 or greater than a full length of central flow tube 1812 such that inflatable contrast balloon 1820 extends beyond an outer extent of proximal arm 1810A and distal arm 1808A along central flow tube 1812. Inflatable contrast balloon 1820 is configured to expand when the contrast agent is injected into inflatable contrast balloon 1820 via contrast tube 1802. When shunt device 1806 is properly deployed (e.g., between the left atrium LA and coronary sinus CS), inflatable contrast balloon 1820 spans both sides of or across tissue wall TW, such that a portion of inflatable contrast balloon 1820 is positioned in the left atrium LA and a portion of inflatable contrast balloon 1820 is positioned in the coronary sinus CS. Inflatable contrast balloon 1820 is sandwiched between tissue wall TW and central flow tube 1812 when shunt device 1806 is properly positioned. Inflatable contrast tube 1802 can be immediately adjacent to tissue wall TW when shunt device 1806 is properly positioned. As contrast balloon 1820 expands with the injection of the contrast agent, inflatable contrast balloon 1820 is pressed inward by tissue wall TW. As illustrated in FIG. 19A, inflatable contrast balloon 1820 can have a kidney bean shape when shunt device 1806 is properly positioned. Tissue wall TW press a center portion of inflatable contrast balloon 1820 toward central flow tube 1812 causing inflatable contrast balloon 1820 to bulge outward on opposite ends (i.e., into the left atrium LA and coronary sinus CS). Inflatable contrast balloon 1820 can be formed of a low pressure compliant material known in the art for medical application and capable of conforming to tissue wall TW without rupture by forces exerted on inflatable contrast balloon 1820 by tissue wall TW or shunt device 1806.

A region including shunt device 1806 can be imaged via fluoroscopy during or following injection of the contrast agent into inflatable contrast balloon 1820. When shunt device 1806 is properly positioned, the kidney bean shape of inflatable contrast balloon 1820 is visible. For example, dark regions indicating the presence of contrast contained in inflatable contrast balloon 1820 are interrupted by a light region indicating the presence of tissue wall TW. **In** other words, the contrast agent is visible on opposite sides of tissue wall TW when shunt device 1806 is properly positioned. If shunt device 1806 is improperly positioned, inflatable contrast balloon 1820 expands between proximal arm 1810A and distal arm 1808A as illustrated in FIG. 19B. Inflatable contrast balloon 1820 can fill a space between proximal arm 1810A and distal arm 1808A when shunt device 1806 is improperly positioned, such that a generally unimpeded ball-like shape of the contrast agent contained in inflatable contrast balloon 1820 may be visible between proximal arm 1810A and distal arm 1808A.

Following imaging, contrast agent can be extracted or withdrawn from inflatable contrast balloon 1820 via contrast tube 1802 and inflatable contrast balloon 1820 can be deflated as illustrated in FIG. 19C. Contrast tube 1802 with inflatable contrast balloon 1820 in a deflated state can then be removed from lumen 1816.

Inflatable contrast balloon 1820 can be used to deliver a contrast agent from an extended location along a length of central flow tube 1812 to direct the contrast agent via expansion of inflatable contrast balloon 1820 to multiple target locations, including, for example, both left atrium LA and coronary sinus CS, to improve confirmation of tissue capture of shunt device 1806 following deployment. Inflatable contrast balloon 1820 is positioned outside of the central flow tube 1812 to allow inflatable contrast balloon 1820 to expand unimpeded by central flow tube 1812. The contrast agent is contained in inflatable contrast balloon 1820 and confined to inflatable contrast balloon 1820 (and contrast tube 1802) such that no contrast agent is released or infused into the human body. As such, delivery device 1800 is particularly important for procedures involving patients in which exposure to the contrast agent is contraindicated.

FIG. 20 is a perspective view of shunt device 1826 for use with delivery devices 1700 and 1800 of FIGS. 17 and 19A-19C. Shunt device 1826 is one of multiple examples of shunt devices that can be used with delivery devices 1700 and 1800 and is provided to illustrate the types of modifications that can be made to shunt device 100 of FIGS. 3A, 3B, and 4 to accommodate contrast tube 1702 and inflatable contrast balloon 1820 of delivery devices 1700 and 1800, respectively. Shunt device 1826, central flow tube 1828, proximal arms 1830A and 1830B, struts 1832, terminal end 1834, opening 1836, first distal arm 1838, and split distal arms 1840A and 1840B are shown. Proximal arm 1830B, distal arm 1838, and central flow tube 1828 can be substantially similar to proximal arm 132B, distal arm 130B, and central flow tube 110 of shunt device 100 illustrated in FIG. 3A. Proximal arm 1830A is disposed on a side of central flow tube 1828 opposite proximal arm 1830B. Distal arms 1840A and 1840B are split apart or separated and are disposed on a side of central flow tube 1828 opposite distal arm 1838. Distal arms 1840A and 1840B are disposed on the same side of central flow tube 1828 as proximal arm 1830A.

Proximal arm 1830A includes struts 1832, which extend from central flow tube 1828 to terminal end 1834, forming opening 1836 therebetween. Opening 1836 is configured to receive contrast tube 1702 or inflatable contrast balloon 1820 of delivery devices 1700 and 1800, respectively. Contrast tube 1702 and inflatable contrast balloon 1820 can be disposed through struts1832 to allow location of contrast tube 1702 and inflatable contrast balloon 1820 along central flow tube 1828. Distal arm 1840A is disposed adjacent to distal arm 1840B. Distal arms 1840A and 1840B separately extend from central flow tube 1828 forming a gap therebetween aligned along central flow tube 1828 and with opening 1836. The gap formed between distal arms 1840A and 1840B is configured to accommodate contrast tube 1702 or contrast balloon 1820 and allow positioning of contrast tube 1702 and inflatable contrast balloon 1820 along an exterior surface of central flow tube 1828. The gap between distal arms 1840A and 1840B and opening 1836 can be sized to accommodate expansion of inflatable contrast balloon 1820.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc. The method of the invention is defined by claim 11.

While the invention has been described with reference to an exemplary embodiment(s), it will be understood by those skilled in the art that various changes may be made without departing from the scope of the invention. Therefore, it is intended that the invention not be limited to the particular embodiment(s) disclosed, but that the invention will include all embodiments falling within the scope of the appended claims.

## Claims

1. A contrast delivery system (1500) for determining tissue capture of a shunt device (1506), the contrast delivery system comprising:
a catheter (1504) comprising:
a lumen (1522) having a distal end;
an exit hole (1526) connecting the lumen to an exterior surface of the catheter; and
a side opening (1528) adjacent to the distal end of the lumen; and
a tube (1502) slidably receivable in the lumen and configured to deliver a contrast agent, the tube comprising:
a closed distal terminal end (1518);
a first distal hole (1514A); and
a proximal hole (1516), the first distal hole and proximal hole spaced along a length of the tube, wherein the first distal hole is disposed between the proximal hole and the closed distal terminal end;
wherein the tube is configured to slide in the lumen between a plurality of positions;
wherein the first distal hole is aligned with the exit hole of the catheter and open to allow flow of the contrast agent through first distal hole and the exit hole when the tube is in a first position; and
wherein the first distal hole is positioned in the side opening and open to allow flow of the contrast agent through the first distal hole and the side opening when the tube is in a second position.

2. The contrast delivery system of claim 1, wherein the proximal hole:
a) is disposed in the lumen when the tube is in the first and second positions; or
b) abuts a wall of the lumen when the tube is in the first position; or
c) is aligned with the exit hole of the catheter and open to allow flow of the contrast agent through the proximal hole and the exit hole when the tube is in the second position; or
d) abuts a wall of the lumen when the tube is in a third position and wherein the first distal hole is open and positioned in the side opening of the catheter when the tube is in the third position, optionally wherein the tube comprises a second distal hole (1514B) disposed between the proximal hole and the first distal hole, wherein the second distal hole is positioned in the side opening of the catheter and open to allow flow of the contrast agent through the second distal hole and the side opening when the tube is in the second and third positions.

3. The contrast delivery system of claim 1 or claim 2, wherein the tube comprises a distal tip (1562), the distal tip including the first distal hole and having a curved shape when the distal tip is positioned in the side opening.

4. The contrast delivery system of any preceding claim, wherein the distal tip comprises a flexible shape-memory material.

5. The contrast delivery system of any preceding claim, wherein:
a) the tube including the distal tip is a nitinol hypotube; or
b) the distal tip further comprises a sleeve, the sleeve having a first hole aligned with the first distal hole; or
c) the distal tip comprises a primary lumen (1564) configured to deliver the contrast agent and a secondary lumen (1566) containing a flexible shape-memory material.

6. The contrast delivery system of any preceding claim, wherein the first distal hole and the proximal hole are aligned along the length of the tube.

7. The contrast delivery system of any preceding claim, wherein the exit hole and side opening are:
a) arranged on the same side of the catheter; or
b) open to an exterior of the catheter.

8. The contrast delivery system of any preceding claim, wherein the distal end of the lumen opens to the side opening.

9. The contrast delivery system of any preceding claim, wherein the lumen is one of a plurality of lumens in the catheter.

10. The contrast delivery system of any preceding claim, wherein the catheter is configured to seat the shunt device, and wherein the shunt device is a blood flow shunt comprising a distal arm (1508A) and a proximal arm (1510A) configured to capture adjacent walls of tissue therebetween, and wherein the first distal hole is directed toward the distal arm and the proximal hole is directed toward the proximal arm when the tube is in the second position.

11. A method for determining tissue capture of a shunt device, the shunt device having a flow tube, proximal arm, and distal arm, the proximal arm and distal arm configured to capture tissue therebetween, the method comprising:
positioning, with a catheter, the shunt device within a human body;
positioning a tube in one of a plurality of positions in the catheter, wherein the tube comprises:
a closed distal terminal end;
a first distal hole; and
a proximal hole, the first distal hole and proximal hole spaced along a length of the tube, wherein the first distal hole is positioned between the proximal hole and the closed distal terminal end;
injecting a contrast agent through the first distal hole when the tube is in a first position, wherein the proximal hole is closed when the tube is in the first position;
advancing or retracting the tube to a second position; and
injecting the contrast agent through the first distal hole and the proximal hole when the tube is in a second position;
wherein the method is performed on a non-living simulation.

12. The method of claim 11, wherein:
a) the catheter comprises an exit hole and wherein the proximal hole is aligned with and open to the exit hole when the tube is in the second position; and/or
b) the method further comprises positioning the tube in a third position, wherein the proximal hole is closed when the tube is in a third position and wherein the first distal hole is open and positioned in a side opening of the catheter when the tube is in the third position.

13. A contrast delivery system (1600) for determining tissue capture of a shunt device (1606), the shunt device having a flow tube (1612), proximal arm (1610A), and distal arm (1608A), the proximal arm and distal arm configured to capture tissue therebetween, the contrast delivery system comprising:
a catheter (1614) comprising:
a lumen (1616);
a channel (1602) extending along a length of the catheter and open to an outer surface of the catheter; and
a feed port (1620) connecting the lumen to the channel, wherein the lumen, channel, and feed port are configured to deliver a contrast agent;
wherein the catheter is positioned through the flow tube and wherein the flow tube is substantially aligned with the channel.

14. A contrast delivery system (1800) for determining tissue capture of a shunt device (1806), the shunt device having a flow tube (1812), proximal arm (1810A), and distal arm (1808A), the proximal arm and distal arm configured to capture tissue therebetween, the contrast delivery system comprising:
a catheter (1804) configured to deliver the shunt device, the catheter comprising:
a lumen (1816); and
a side opening (1818); and
a tube (1802) received in the lumen and configured to deliver a contrast agent, the tube comprising an inflatable balloon (1820) on a distal end, wherein the balloon is disposed adjacent to the side opening and an exterior surface of the flow tube of the shunt device.

15. A contrast delivery system (1700) for determining tissue capture of a shunt device (1706), the shunt device having a flow tube (1712), proximal arm (1710A), and distal arm (1708A), the proximal arm and distal arm configured to capture tissue therebetween, the contrast delivery system comprising:
a catheter (1704) configured to deliver the shunt device, the catheter comprising:
a lumen (1716); and
a side opening (1720); and
a tube (1702) receivable in the lumen and configured to deliver a contrast agent, the tube comprising:
a closed distal terminal end (1718); and
a slit opening (1724) extending along a length of the tube, wherein a portion of the tube including the slit opening is disposable adjacent to an exterior surface of the flow tube of the shunt device and wherein the slit opening faces away from the exterior surface of the flow tube.

## Patentansprüche

1. Kontrastmittelabgabesystem (1500) zum Bestimmen der Gewebeerfassung einer Shuntvorrichtung (1506), das Kontrastmittelabgabesystem umfassend:
einen Katheter (1504), umfassend:
ein Lumen (1522) mit einem distalen Ende;
ein Austrittsloch (1526), das das Lumen mit einer Außenoberfläche des Katheters verbindet; und
eine Seitenöffnung (1528) angrenzend an das distale Ende des Lumens; und
einen Schlauch (1502), der verschiebbar in dem Lumen aufgenommen werden kann und dazu ausgelegt ist, ein Kontrastmittel abzugeben,
der Schlauch umfassend:
ein geschlossenes distales Abschlussende (1518);
ein erstes distales Loch (1514A); und
ein proximales Loch (1516), wobei das erste distale Loch und das proximale Loch entlang einer Länge des Schlauchs beabstandet sind, wobei das erste distale Loch zwischen dem proximalen Loch und dem geschlossenen distalen Abschlussende angeordnet ist;
wobei der Schlauch dazu ausgelegt ist, in dem Lumen zwischen einer Vielzahl von Positionen zu gleiten;
wobei das erste distale Loch an dem Austrittsloch des Katheters ausgerichtet und offen ist, um einen Fluss des Kontrastmittels durch das erste distale Loch und das Austrittsloch zu ermöglichen, wenn sich der Schlauch in einer ersten Position befindet; und
wobei das erste distale Loch in der Seitenöffnung positioniert und offen ist, um einen Fluss des Kontrastmittels durch das erste distale Loch und die Seitenöffnung zu ermöglichen, wenn sich der Schlauch in einer zweiten Position befindet.

2. Kontrastmittelabgabesystem nach Anspruch 1, wobei das proximale Loch:
a) in dem Lumen angeordnet ist, wenn sich der Schlauch in der ersten und der zweiten Position befindet; oder
b) an einer Wand des Lumens anliegt, wenn sich der Schlauch in der ersten Position befindet; oder
c) an dem Austrittsloch des Katheters ausgerichtet und offen ist, um einen Fluss des Kontrastmittels durch das proximale Loch und das Austrittsloch zu ermöglichen, wenn sich der Schlauch in der zweiten Position befindet; oder
d) an eine Wand des Lumens stößt, wenn sich der Schlauch in einer dritten Position befindet, und wobei das erste distale Loch offen und in der Seitenöffnung des Katheters positioniert ist, wenn sich der Schlauch in der dritten Position befindet, wobei der Schlauch optional ein zweites distales Loch (1514B) umfasst, das zwischen dem proximalen Loch und dem ersten distalen Loch angeordnet ist, wobei das zweite distale Loch in der Seitenöffnung des Katheters positioniert und offen ist, um einen Fluss des Kontrastmittels durch das zweite distale Loch und die Seitenöffnung zu ermöglichen, wenn sich der Schlauch in der zweiten und der dritten Position befindet.

3. Kontrastmittelabgabesystem nach Anspruch 1 oder 2, wobei der Schlauch eine distale Spitze (1562) umfasst, wobei die distale Spitze das erste distale Loch beinhaltet und eine gekrümmte Form aufweist, wenn die distale Spitze in der Seitenöffnung positioniert ist.

4. Kontrastmittelabgabesystem nach einem der vorhergehenden Ansprüche, wobei die distale Spitze ein flexibles Formgedächtnismaterial umfasst.

5. Kontrastmittelabgabesystem nach einem der vorhergehenden Ansprüche, wobei:
a) der Schlauch einschließlich der distalen Spitze ein Nitinol-Hyposchlauch ist; oder
b) die distale Spitze ferner eine Hülse umfasst, wobei die Hülse ein erstes Loch aufweist, das an dem ersten distalen Loch ausgerichtet ist; oder
c) die distale Spitze ein Primärlumen (1564), ausgelegt zum Zuführen des Kontrastmittels, und ein Sekundärlumen (1566), das ein flexibles Formgedächtnismaterial beinhaltet, umfasst.

6. Kontrastmittelabgabesystem nach einem der vorhergehenden Ansprüche, wobei das erste distale Loch und das proximale Loch entlang der Länge des Schlauchs ausgerichtet sind.

7. Kontrastmittelabgabesystem nach einem der vorhergehenden Ansprüche, wobei das Austrittsloch und die Seitenöffnung:
a) auf derselben Seite des Katheters angeordnet sind; oder
b) zu einem Äußeren des Katheters hin offen sind.

8. Kontrastmittelabgabesystem nach einem der vorhergehenden Ansprüche, wobei sich das distale Ende des Lumens zu der Seitenöffnung hin öffnet.

9. Kontrastmittelabgabesystem nach einem der vorhergehenden Ansprüche, wobei das Lumen eines von einer Vielzahl von Lumen in dem Katheter ist.

10. Kontrastmittelabgabesystem nach einem der vorhergehenden Ansprüche, wobei der Katheter dazu ausgelegt ist, die Shuntvorrichtung zu setzen, und wobei die Shuntvorrichtung ein Blutfluss-Shunt ist, umfassend einen distalen Arm (1508A) und einen proximalen Arm (1510A), die dazu ausgelegt sind, benachbarte Wände von Gewebe dazwischen zu erfassen, und wobei das erste distale Loch zum distalen Arm hin gerichtet ist und das proximale Loch zum proximalen Arm hin gerichtet ist, wenn sich der Schlauch in der zweiten Position befindet.

11. Verfahren zum Bestimmen der Gewebeerfassung einer Shuntvorrichtung, wobei die Shuntvorrichtung einen Durchflussschlauch, einen proximalen Arm und einen distalen Arm aufweist, wobei der proximale Arm und der distale Arm dazu ausgelegt sind, Gewebe dazwischen zu erfassen, das Verfahren umfassend:
Positionieren, mit einem Katheter, der Shuntvorrichtung innerhalb eines menschlichen Körpers;
Positionieren eines Schlauchs in einer von einer Vielzahl von Positionen in dem Katheter, wobei der Schlauch umfasst:
ein geschlossenes distales Anschlussende;
ein erstes distales Loch; und
ein proximales Loch, wobei das erste distale Loch und das proximale Loch entlang einer Länge des Schlauchs beabstandet sind, wobei das erste distale Loch zwischen dem proximalen Loch und dem geschlossenen distalen Abschlussende positioniert ist;
Injizieren eines Kontrastmittels durch das erste distale Loch, wenn sich der Schlauch in einer ersten Position befindet, wobei das proximale Loch geschlossen ist, wenn sich der Schlauch in der ersten Position befindet;
Vorschieben oder Zurückziehen des Schlauchs in eine zweite Position; und
Injizieren des Kontrastmittels durch das erste distale Loch und das proximale Loch, wenn sich der Schlauch in einer zweiten Position befindet;
wobei das Verfahren an einer nicht lebenden Simulation durchgeführt wird.

12. Verfahren nach Anspruch 11, wobei:
a) der Katheter ein Austrittsloch umfasst und wobei das proximale Loch an dem Austrittsloch ausgerichtet und zu diesem offen ist, wenn sich der Schlauch in der zweiten Position befindet; und/oder
b) das Verfahren ferner das Positionieren des Schlauchs in einer dritten Position umfasst, wobei das proximale Loch geschlossen ist, wenn sich der Schlauch in einer dritten Position befindet, und wobei das erste distale Loch offen ist und in einer Seitenöffnung des Katheters positioniert ist, wenn sich der Schlauch in der dritten Position befindet.

13. Kontrastmittelabgabesystem (1600) zum Bestimmen der Gewebeerfassung einer Shuntvorrichtung (1606), wobei die Shuntvorrichtung einen Durchflussschlauch (1612), einen proximalen Arm (1610A) und einen distalen Arm (1608A) aufweist, wobei der proximale Arm und der distale Arm dazu ausgelegt sind, Gewebe dazwischen zu erfassen, das Kontrastmittelabgabesystem umfassend:
einen Katheter (1614), umfassend:
ein Lumen (1616);
einen Kanal (1602), der sich entlang einer Länge des Katheters erstreckt und zu einer Außenoberfläche des Katheters hin offen ist; und
einen Zuführanschluss (1620), der das Lumen mit dem Kanal verbindet, wobei das Lumen, der Kanal und der Zuführanschluss dazu ausgelegt sind, ein Kontrastmittel abzugeben;
wobei der Katheter durch den Durchflussschlauch hindurch positioniert ist und wobei der Durchflussschlauch im Wesentlichen an dem Kanal ausgerichtet ist.

14. Kontrastmittelabgabesystem (1800) zum Bestimmen der Gewebeerfassung einer Shuntvorrichtung (1806), wobei die Shuntvorrichtung einen Durchflussschlauch (1812), einen proximalen Arm (1810A) und einen distalen Arm (1808A) aufweist, wobei der proximale Arm und der distale Arm dazu ausgelegt sind, Gewebe dazwischen zu erfassen, das Kontrastmittelabgabesystem umfassend:
einen Katheter (1804), dazu ausgelegt, die Shuntvorrichtung zuzuführen, der Katheter umfassend:
ein Lumen (1816); und
eine Seitenöffnung (1818); und
einen Schlauch (1802), der in dem Lumen aufgenommen wird und dazu ausgelegt ist, ein Kontrastmittel abzugeben, der Schlauch umfassend einen aufblasbaren Ballon (1820) an einem distalen Ende, wobei der Ballon angrenzend an die Seitenöffnung und eine Außenoberfläche des Durchflussschlauchs der Shuntvorrichtung angeordnet ist.

15. Kontrastmittelabgabesystem (1700) zum Bestimmen der Gewebeerfassung einer Shuntvorrichtung (1706), wobei die Shuntvorrichtung einen Durchflussschlauch (1712), einen proximalen Arm (1710A) und einen distalen Arm (1708A) aufweist, wobei der proximale Arm und der distale Arm dazu ausgelegt sind, Gewebe dazwischen zu erfassen, das Kontrastmittelabgabesystem umfassend:
einen Katheter (1704), dazu ausgelegt, die Shuntvorrichtung zuzuführen, der Katheter umfassend:
ein Lumen (1716); und
eine Seitenöffnung (1720); und
einen Schlauch (1702), der in dem Lumen aufgenommen werden kann und dazu ausgelegt ist, ein Kontrastmittel abzugeben, der Schlauch umfassend:
ein geschlossenes distales Abschlussende (1718); und
eine Schlitzöffnung (1724), die sich entlang einer Länge des Schlauchs erstreckt, wobei ein Abschnitt des Schlauchs, der die Schlitzöffnung beinhaltet, angrenzend an eine Außenoberfläche des Durchflussschlauchs der Shuntvorrichtung angeordnet werden kann, und wobei die Schlitzöffnung von der Außenoberfläche des Durchflussschlauchs weg weist.

## Revendications

1. Système d'administration de contraste (1500) pour déterminer la capture de tissu d'un dispositif de dérivation (1506), le système d'administration de contraste comprenant :
un cathéter (1504) comprenant :
une lumière (1522) ayant une extrémité distale ;
un orifice de sortie (1526) reliant la lumière à une surface extérieure du cathéter ; et
une ouverture latérale (1528) adjacente à l'extrémité distale de la lumière ; et
un tube (1502) pouvant être reçu de manière coulissante dans la lumière et configuré pour administrer un agent de contraste,
le tube comprenant :
une extrémité terminale distale fermée (1518) ;
un premier orifice distal (1514A) ; et
un orifice proximal (1516), le premier orifice distal et l'orifice proximal espacés le long d'une longueur du tube, dans lequel le premier orifice distal est disposé entre l'orifice proximal et l'extrémité terminale distale fermée ;
dans lequel le tube est configuré pour coulisser dans la lumière entre une pluralité de positions ;
dans lequel le premier orifice distal est aligné avec l'orifice de sortie du cathéter et ouvert pour permettre l'écoulement de l'agent de contraste à travers le premier orifice distal et l'orifice de sortie lorsque le tube est dans une première position ; et
dans lequel le premier orifice distal est positionné dans l'ouverture latérale et ouvert pour permettre l'écoulement de l'agent de contraste à travers le premier orifice distal et l'ouverture latérale lorsque le tube est dans une deuxième position.

2. Système d'administration de contraste selon la revendication 1, dans lequel l'orifice proximal :
a) est disposé dans la lumière lorsque le tube est dans les première et deuxième positions ; ou
b) bute contre une paroi de la lumière lorsque le tube est dans la première position ; ou
c) est aligné avec l'orifice de sortie du cathéter et ouvert pour permettre l'écoulement de l'agent de contraste à travers l'orifice proximal et l'orifice de sortie lorsque le tube est dans la deuxième position ; ou
d) bute contre une paroi de la lumière lorsque le tube est dans une troisième position et dans lequel le premier orifice distal est ouvert et positionné dans l'ouverture latérale du cathéter lorsque le tube est dans la troisième position, facultativement dans lequel le tube comprend un deuxième orifice distal (1514B) disposé entre l'orifice proximal et le premier orifice distal, dans lequel le deuxième orifice distal est positionné dans l'ouverture latérale du cathéter et ouvert pour permettre l'écoulement de l'agent de contraste à travers le deuxième orifice distal et l'ouverture latérale lorsque le tube est dans les deuxième et troisième positions.

3. Système d'administration de contraste selon la revendication 1 ou la revendication 2, dans lequel le tube comprend une pointe distale (1562), la pointe distale incluant le premier orifice distal et ayant une forme incurvée lorsque la pointe distale est positionnée dans l'ouverture latérale.

4. Système d'administration de contraste selon l'une quelconque des revendications précédentes, dans lequel la pointe distale comprend un matériau à mémoire de forme flexible.

5. Système d'administration de contraste selon l'une quelconque des revendications précédentes, dans lequel :
a) le tube incluant la pointe distale est un hypotube de nitinol ; ou
b) la pointe distale comprend en outre un manchon, le manchon ayant un premier orifice aligné avec le premier orifice distal ; ou
c) la pointe distale comprend une lumière primaire (1564) configurée pour administrer l'agent de contraste et une lumière secondaire (1566) contenant un matériau à mémoire de forme flexible.

6. Système d'administration de contraste selon l'une quelconque des revendications précédentes, dans lequel le premier orifice distal et l'orifice proximal sont alignés le long de la longueur du tube.

7. Système d'administration de contraste selon l'une quelconque des revendications précédentes, dans lequel l'orifice de sortie et l'ouverture latérale sont :
a) agencés du même côté du cathéter ; ou
b) ouverts sur un extérieur du cathéter.

8. Système d'administration de contraste selon l'une quelconque des revendications précédentes, dans lequel l'extrémité distale de la lumière s'ouvre sur l'ouverture latérale.

9. Système d'administration de contraste selon l'une quelconque des revendications précédentes, dans lequel la lumière est l'une d'une pluralité de lumières dans le cathéter.

10. Système d'administration de contraste selon l'une quelconque des revendications précédentes, dans lequel le cathéter est configuré pour asseoir le dispositif de dérivation, et dans lequel le dispositif de dérivation est un shunt d'écoulement sanguin comprenant un bras distal (1508A) et un bras proximal (1510A) configurés pour capturer des parois adjacentes de tissu entre eux, et dans lequel le premier orifice distal est dirigé vers le bras distal et l'orifice proximal est dirigé vers le bras proximal lorsque le tube est dans la deuxième position.

11. Procédé pour déterminer la capture de tissu d'un dispositif de dérivation, le dispositif de dérivation ayant un tube d'écoulement, un bras proximal, et un bras distal, le bras proximal et le bras distal configurés pour capturer du tissu entre eux, le procédé comprenant :
le positionnement, avec un cathéter, du dispositif de dérivation dans un corps humain ;
le positionnement d'un tube dans l'une d'une pluralité de positions dans le cathéter, dans lequel le tube comprend :
une extrémité terminale distale fermée ;
un premier orifice distal ; et
un orifice proximal, le premier orifice distal et l'orifice proximal espacés le long d'une longueur du tube, dans lequel le premier orifice distal est positionné entre l'orifice proximal et l'extrémité terminale distale fermée ;
l'injection d'un agent de contraste à travers le premier orifice distal lorsque le tube est dans une première position, dans lequel l'orifice proximal est fermé lorsque le tube est dans la première position ;
l'avance ou la rétraction du tube dans une deuxième position ; et
l'injection de l'agent de contraste à travers le premier orifice distal et l'orifice proximal lorsque le tube est dans une deuxième position ;
le procédé étant réalisé sur une simulation non vivante.

12. Procédé selon la revendication 11, dans lequel :
a) le cathéter comprend un orifice de sortie et dans lequel l'orifice proximal est aligné avec et ouvert sur l'orifice de sortie lorsque le tube est dans la deuxième position ; et/ou
b) le procédé comprend en outre le positionnement du tube dans une troisième position, dans lequel l'orifice proximal est fermé lorsque le tube est dans une troisième position et dans lequel le premier orifice distal est ouvert et positionné dans une ouverture latérale du cathéter lorsque le tube est dans la troisième position.

13. Système d'administration de contraste (1600) pour déterminer la capture de tissu d'un dispositif de dérivation (1606), le dispositif de dérivation ayant un tube d'écoulement (1612), un bras proximal (1610A), et un bras distal (1608A), le bras proximal et le bras distal configurés pour capturer du tissu entre eux, le système d'administration de contraste comprenant :
un cathéter (1614) comprenant :
une lumière (1616) ;
un canal (1602) s'étendant le long d'une longueur du cathéter et ouvert sur une surface externe du cathéter ; et
un orifice d'alimentation (1620) reliant la lumière au canal, dans lequel la lumière, le canal, et l'orifice d'alimentation sont configurés pour administrer un agent de contraste ;
dans lequel le cathéter est positionné à travers le tube d'écoulement et dans lequel le tube d'écoulement est substantiellement aligné avec le canal.

14. Système d'administration de contraste (1800) pour déterminer la capture de tissu d'un dispositif de dérivation (1806), le dispositif de dérivation ayant un tube d'écoulement (1812), un bras proximal (1810A), et un bras distal (1808A), le bras proximal et le bras distal étant configurés pour capturer du tissu entre eux, le système d'administration de contraste comprenant :
un cathéter (1804) configuré pour administrer le dispositif de dérivation, le cathéter comprenant :
une lumière (1816) ; et
une ouverture latérale (1818) ; et
un tube (1802) reçu dans la lumière et configuré pour administrer un agent de contraste, le tube comprenant un ballon gonflable (1820) sur une extrémité distale, dans lequel le ballon est disposé adjacent à l'ouverture latérale et à une surface extérieure du tube d'écoulement du dispositif de dérivation.

15. Système d'administration de contraste (1700) pour déterminer la capture de tissu d'un dispositif de dérivation (1706), le dispositif de dérivation ayant un tube d'écoulement (1712), un bras proximal (1710A), et un bras distal (1708A), le bras proximal et le bras distal configurés pour capturer du tissu entre eux, le système d'administration de contraste comprenant :
un cathéter (1704) configuré pour administrer le dispositif de dérivation, le cathéter comprenant :
une lumière (1716) ; et
une ouverture latérale (1720) ; et
un tube (1702) pouvant être reçu dans la lumière et configuré pour administrer un agent de contraste, le tube comprenant :
une extrémité terminale distale fermée (1718) ; et
une ouverture en fente (1724) s'étendant le long d'une longueur du tube, dans lequel une partie du tube incluant l'ouverture en fente peut être disposée adjacente à une surface extérieure du tube d'écoulement du dispositif de dérivation et dans lequel l'ouverture en fente est orientée à l'écart de la surface extérieure du tube d'écoulement.
